# EUROPÄISCHE PATENTSCHRIFT

(11) **EP 1 570 060 B1**
(45) Veröffentlichungstag und Bekanntmachung des Hinweises auf die Patenterteilung: **01.08.2007**
(21) Anmeldenummer: 03780076.0
(22) Anmeldetag: 27.11.2003
(51) Int. Cl.: C12N 15/63

(54) **L-RHAMNOSE-INDUZIERBARE EXPRESSIONSSYSTEME**
L-RHAMNOSE-INDUCIBLE EXPRESSION SYSTEMS
SYSTEMES D'EXPRESSION POUVANT ETRE INDUITS PAR LE L-RHAMNOSE

(30) Priorität: 02.12.2002 DE 10256381
(43) Veröffentlichungstag der Anmeldung: 07.09.2005
(73) Patentinhaber: BASF Aktiengesellschaft, 67056 Ludwigshafen (DE)
(72) Erfinder: KESSELER, Maria, 68167 Mannheim (DE); ZELINSKI, Thomas, 67271 Neuleiningen (DE); HAUER, Bernhard, 67136 Fussgönheim (DE)
(86) Internationale Anmeldenummer: PCT/EP2003/013367
(87) Internationale Veröffentlichungsnummer: WO 2004/050877

(56) Entgegenhaltungen:
- STUMPP T ET AL: "EIN NEUES, L-RHAMNOSE-INDUZIERBARES EXPRESSIONSSYSTEM FUER ESCHERICHIA COLI" BIOSPEKTRUM, SPEKTRUM AKADEMISCHER VERLAG, DE, Bd. 6, Nr. 1, 2000, Seiten 33-36, XP009004621 ISSN: 0947-0867 in der Anmeldung erwähnt
- WILMS B ET AL: "High-cell-density fermentation for production of L-N-carbamoylase using an expression system based on the Escherichia coli rhaBAD promoter" BIOTECHNOLOGY AND BIOENGINEERING. INCLUDING: SYMPOSIUM BIOTECHNOLOGY IN ENERGY PRODUCTION AND CONSERVATION, JOHN WILEY & SONS. NEW YORK, US, Bd. 73, Nr. 2, 20. April 2001 (2001-04-20), Seiten 95-103, XP002228440 ISSN: 0006-3592 in der Anmeldung erwähnt
- BULAWA C E ET AL: "ISOLATION AND CHARACTERIZATION OF ESCHERICHIA-COLI STRAINS DEFECTIVE IN CDP-DIGLYCERIDE HYDROLASE" JOURNAL OF BIOLOGICAL CHEMISTRY, Bd. 259, Nr. 18, 1984, Seiten 11257-11264, XP002272208 ISSN: 0021-9258 in der Anmeldung erwähnt
- HALDIMANN ANDREAS ET AL: "Use of new methods for construction of tightly regulated arabinose and rhamnose promoter fusions in studies of the Escherichia coli phosphate regulon" JOURNAL OF BACTERIOLOGY, Bd. 180, Nr. 5, März 1998 (1998-03), Seiten 1277-1286, XP002272209 ISSN: 0021-9193 in der Anmeldung erwähnt
- EGAN SUSAN M ET AL: "A regulatory cascade in the induction of rhaBAD" JOURNAL OF MOLECULAR BIOLOGY, Bd. 234, Nr. 1, 1993, Seiten 87-98, XP002272210 ISSN: 0022-2836 in der Anmeldung erwähnt

### SEQUENZPROTOKOLL

<110> BASF Aktiengesellschaft
   <120> L-Rhamnose-induzierbare Expressionssysteme
   <130> AE20020689
<140>
   <141>
<160> 19
<170> PatentIn Ver. 2.1
<210> 1
   <211> 2046
   <212> DNA
   <213> Escherichia coli
<220>
   <221> misc_feature
   <222> (288)..(1121)
   <223> coding for rhaS (positve regulator of rhaBAD operon)
<220>
   <221> misc_feature
   <222> (1108)..(2043)
   <223> coding for rhaR (positive regulator of rhaRS operon)
<220>
   <221> protein_bind
   <222> (56)..(72)
   <223> potential RhaS binding site
<220>
   <221> protein_bind
   <222> (89)..(105)
   <223> potential RhaS binding site
<220>
   <221> protein_bind
   <222> (172)..(203)
   <223> potential RhaR binding site
<220>
   <221> protein_bind
   <222> (210) ..(241)
   <223> potential RhaR binding site
<220>
   <221> misc_feature
   <222> (24)
   <223> potential start of transcription (complement)
<400> 1
   aatgtgatcc tgctgaattt cattacgacc agtctaaaaa gcgcctgaat tcgcgacctt 60
   ctcgttactg acaggaaaat gggccattgg caaccaggga aagatgaacg tgatgatgtt 120
   cacaatttgc tgaattgtgg tgatgtgatg ctcaccgcat ttcctgaaaa ttcacgctgt 180
   atcttgaaaa atcgacgttt tttacgtggt tttccgtcga aaatttaagg taagaacctg 240
   acctcgtgat tactatttcg ccgtgttgac gacatcagga ggccagtatg accgtattac 300
   atagtgtgga tttttttccg tctggtaacg cgtccgtggc gatagaaccc cggctcccgc 360
   aggcggattt tcctgaacat catcatgatt ttcatgaaat tgtgattgtc gaacatggca 420
   cgggtattca tgtgtttaat gggcagccct ataccatcac cggtggcacg gtctgtttcg 480
   tacgcgatca tgatcggcat ctgtatgaac ataccgataa tctgtgtctg accaatgtgc 540
   tgtatcgctc gccggatcga tttcagtttc tcgccgggct gaatcagttg ctgccacaag 600
   agctggatgg gcagtatccg tctcactggc gcgttaacca cagcgtattg cagcaggtgc 660
   gacagctggt tgcacagatg gaacagcagg aaggggaaaa tgatttaccc tcgaccgcca 720
   gtcgcgagat cttgtttatg caattactgc tcttgctgcg taaaagcagt ttgcaggaga 780
   acctggaaaa cagcgcatca cgtctcaact tgcttctggc ctggctggag gaccattttg 840
   ccgatgaggt gaattgggat gccgtggcgg atcaattttc tctttcactg cgtacgctac 900
   atcggcagct taagcagcaa acgggactga cgcctcagcg atacctgaac cgcctgcgac 960
   tgatgaaagc ccgacatctg ctacgccaca gcgaggccag cgttactgac atcgcctatc 1020
   gctgtggatt cagcgacagt aaccactttt cgacgctttt tcgccgagag tttaactggt 1080
   caccgcgtga tattcgccag ggacgggatg gctttctgca ataacgcgaa tcttctcaac 1140
   gtatttgtac gccatattgc gaataatcaa cttcgttctc tggccgaggt agccacggtg 1200
   gcgcatcagt taaaacttct caaagatgat ttttttgcca gcgaccagca ggcagtcgct 1260
   gtggctgacc gttatccgca agatgtcttt gctgaacata cacatgattt ttgtgagctg 1320
   gtgattgtct ggcgcggtaa tggcctgcat gtactcaacg atcgccctta tcgcattacc 1380
   cgtggcgatc tcttttacat tcatgctgac gataaacact cctacgcttc cgttaacgat 1440
   ctggttttgc agaatattat ttattgcccg gagcgtctga agctgaatct tgactggcag 1500
   ggggcgattc cgggatttaa cgccagcgca gggcaaccac actggcgctt aggtagcatg 1560
   gggatggcgc aggcgcggca ggttatcggt cagcttgagc atgaaagtag tcagcatgtg 1620
   ccgtttgcta acgaaatggc tgagttgctg ttcgggcagt tggtgatgtt gctgaatcgc 1680
   catcgttaca ccagtgattc gttgccgcca acatccagcg aaacgttgct ggataagctg 1740
   attacccggc tggcggctag cctgaaaagt ccctttgcgc tggataaatt ttgtgatgag 1800
   gcatcgtgca gtgagcgcgt tttgcgtcag caatttcgcc agcagactgg aatgaccatc 1860
   aatcaatatc tgcgacaggt cagagtgtgt catgcgcaat atcttctcca gcatagccgc 1920
   ctgttaatca gtgatatttc gaccgaatgt ggctttgaag atagtaacta tttttcggtg 1980
   gtgtttaccc gggaaaccgg gatgacgccc agccagtggc gtcatctcaa ttcgcagaaa 2040
   gattaa                                                            2046
<210> 2
   <211> 287
   <212> DNA
   <213> Escherichia coli
<220>
   <221> promoter
   <222> (1)..(287)
   <223> rhaBAD promoter fragment containing rhaS and rhaR binding sites
<400> 2
   actggcctcc tgatgtcgtc aacacggcga aatagtaatc acgaggtcag gttcttacct 60
   taaattttcg acggaaaacc acgtaaaaaa cgtcgatttt tcaagataca gcgtgaattt 120
   tcaggaaatg cggtgagcat cacatcacca caattcagca aattgtgaac atcatcacgt 180
   tcatctttcc ctggttgcca atggcccatt ttcctgtcag taacgagaag gtcgcgaatt 240
   caggcgcttt ttagactggt cgtaatgaaa ttcagcagga tcacatt              287
<210> 3
   <211> 125
   <212> DNA
   <213> Escherichia coli
<220>
   <221> promoter
   <222> (1) .. (125)
   <223> rhaBAD promoter fragment containing RhaS binding site
<400> 3
   ttgtgaacat catcacgttc atctttccct ggttgccaat ggcccatttt cctgtcagta 60
   acgagaaggt cgcgaattca ggcgcttttt agactggtcg taatgaaatt cagcaggatc 120
   acatt 125
<210> 4
   <211> 123
   <212> DNA
   <213> Escherichia coli
<220>
   <221> promoter
   <222> (1)..(123)
   <223> rhaBAD promoter fragment containing RhaS binding site
<400> 4
   atcaccacaa ttcagcaaat tgtgaacatc atcacgttca tctttccctg gttgccaatg 60
   gcccattttc ctgtcagtaa cgagaaggtc gcgaattcag gcgcttttta gactggtcgt 120
   aat 123
<210> 5
   <211> 51
   <212> DNA
   <213> Escherichia coli
<220>
   <221> misc_feature
   <222> (1)..(51)
   <223> palindromic RhaS binding site of rhaBAD promoter
<400> 5
   atctttccct ggttgccaat ggcccatttt cctgtcagta acgagaaggt c 51
<210> 6
   <211> 1071
   <212> DNA
   <213> Alcaligenes faecalis
<220>
   <221> CDS
   <222> (1)..(1068)
   <223> coding for nitrilase
<400> 6
   atg cag aca aga aaa atc gtc cgg gca gcc gcc gta cag gcc gcc tct      48
   Met Gln Thr Arg Lys Ile Val Arg Ala Ala Ala Val Gln Ala Ala Ser
    1               5                  10                  15
   ccc aac tac gat ctg gca acg ggt gtt gat aaa acc att gag ctg gct      96
   Pro Asn Tyr Asp Leu Ala Thr Gly Val Asp Lys Thr Ile Glu Leu Ala
               20                  25                  30
   cgt cag gcc cgc gat gag ggc tgt gac ctg atc gtg ttt ggt gaa acc     144
   Arg Gln Ala Arg Asp Glu Gly Cys Asp Leu Ile Val Phe Gly Glu Thr
           35                  40                  45
   tgg ctg ccc gga tat ccc ttc cac gtc tgg ctg ggc gca ccg gcc tgg     192
   Trp Leu Pro Gly Tyr Pro Phe His Val Trp Leu Gly Ala Pro Ala Trp
       50                  55                  60
   tcg ctg aaa tac agt gcc cgc tac tat gcc aac tcg ctc tcg ctg gac     240
   Ser Leu Lys Tyr Ser Ala Arg Tyr Tyr Ala Asn Ser Leu Ser Leu Asp
   65                  70                  75                  80
   agt gca gag ttt caa cgc att gcc cag gcc gca cgg acc ttg ggt att     288
   Ser Ala Glu Phe Gln Arg Ile Ala Gln Ala Ala Arg Thr Leu Gly Ile
                   85                  90                  95
   ttc atc gca ctg ggt tat agc gag cgc agc ggc ggc agc ctt tac ctg     336
   Phe Ile Ala Leu Gly Tyr Ser Glu Arg Ser Gly Gly Ser Leu Tyr Leu
               100                 105                 110
   ggc caa tgc ctg atc gac gac aag ggc gag atg ctg tgg tcg cgt cgc      384
   Gly Gln Cys Leu Ile Asp Asp Lys Gly Glu Met Leu Trp Ser Arg Arg
           115                 120                 125
   aaa ctc aaa ccc acg cat gta gag cgc acc gta ttt ggt gaa ggt tat      432
   Lys Leu Lys Pro Thr His Val Glu Arg Thr Val Phe Gly Glu Gly Tyr
       130                 135                 140
   gcc cgt gat ctg att gtg tcc gac aca gaa ctg gga cgc gtc ggt gct      480
   Ala Arg Asp Leu Ile Val Ser Asp Thr Glu Leu Gly Arg Val Gly Ala
   145                 150                 155                 160
   cta tgc tgc tgg gag cat ttg tcg ccc ttg agc aag tac gcg ctg tac      528
   Leu Cys Cys Trp Glu His Leu Ser Pro Leu Ser Lys Tyr Ala Leu Tyr
                   165                 170                 175
   tcc cag cat gaa gcc att cac att gct gcc tgg ccg tcg ttt tcg cta      576
   Ser Gln His Glu Ala Ile His Ile Ala Ala Trp Pro Ser Phe Ser Leu
               180                 185                 190
   tac agc gaa cag gcc cac gcc ctc agt gcc aag gtg aac atg gct gcc      624
   Tyr Ser Glu Gln Ala His Ala Leu Ser Ala Lys Val Asn Met Ala Ala
           195                 200                 205
   tcg caa atc tat tcg gtt gaa ggc cag tgc ttt acc atc gcc gcc agc      672
   Ser Gln Ile Tyr Ser Val Glu Gly Gln Cys Phe Thr Ile Ala Ala Ser
       210                 215                 220
   agt gtg gtc acc caa gag acg cta gac atg ctg gaa gtg ggt gaa cac      720
   Ser Val Val Thr Gln Glu Thr Leu Asp Met Leu Glu Val Gly Glu His
   225                 230                 235                 240
   aac gcc ccc ttg ctg aaa gtg ggc ggc ggc agt tcc atg att ttt gcg      768
   Asn Ala Pro Leu Leu Lys Val Gly Gly Gly Ser Ser Met Ile Phe Ala
                   245                 250                 255
   ccg gac gga cgc aca ctg gct ccc tac ctg cct cac gat gcc gag ggc      816
   Pro Asp Gly Arg Thr Leu Ala Pro Tyr Leu Pro His Asp Ala Glu Gly
               260                 265                 270
   ttg atc att gcc gat ctg aat atg gag gag att gcc ttc gcc aaa gcg      864
   Leu Ile Ile Ala Asp Leu Asn Met Glu Glu Ile Ala Phe Ala Lys Ala
           275                 280                 285
   atc aat gac ccc gta ggc cac tat tcc aaa ccc gag gcc acc cgt ctg      912
   Ile Asn Asp Pro Val Gly His Tyr Ser Lys Pro Glu Ala Thr Arg Leu
       290                 295                 300
   gtg ctg gac ttg ggg cac cga gac ccc atg act cgg gtg cac tcc aaa      960
   Val Leu Asp Leu Gly His Arg Asp Pro Met Thr Arg Val His Ser Lys
   305                 310                 315                 320
   agc gtg acc agg gaa gag gct ccc gag caa ggt gtg caa agc aag att     1008
   Ser Val Thr Arg Glu Glu Ala Pro Glu Gln Gly Val Gln Ser Lys Ile
                   325                 330                 335
   gcc tca gtc gct atc agc cat cca cag gac tcg gac aca ctg cta gtg     1056
   Ala Ser Val Ala Ile Ser His Pro Gln Asp Ser Asp Thr Leu Leu Val
               340                 345                 350
   caa gag ccg tct tga                                                 1071
   Gln Glu Pro Ser
           355
<210> 7
   <211> 356
   <212> PRT
   <213> Alcaligenes faecalis
<400> 7
   Met Gln Thr Arg Lys Ile Val Arg Ala Ala Ala Val Gln Ala Ala Ser
   1 5 10 15
   Pro Asn Tyr Asp Leu Ala Thr Gly Val Asp Lys Thr Ile Glu Leu Ala
   20 25 30
   Arg Gln Ala Arg Asp Glu Gly Cys Asp Leu Ile Val Phe Gly Glu Thr
   35 40 45
   Trp Leu Pro Gly Tyr Pro Phe His Val Trp Leu Gly Ala Pro Ala Trp
   50 55 60
   Ser Leu Lys Tyr Ser Ala Arg Tyr Tyr Ala Asn Ser Leu Ser Leu Asp
   65 70 75 80
   Ser Ala Glu Phe Gln Arg Ile Ala Gln Ala Ala Arg Thr Leu Gly Ile
   85 90 95
   Phe Ile Ala Leu Gly Tyr Ser Glu Arg Ser Gly Gly Ser Leu Tyr Leu
   100 105 110
   Gly Gln Cys Leu Ile Asp Asp Lys Gly Glu Met Leu Trp Ser Arg Arg
   115 120 125
   Lys Leu Lys Pro Thr His Val Glu Arg Thr Val Phe Gly Glu Gly Tyr
   130 135 140
   Ala Arg Asp Leu Ile Val Ser Asp Thr Glu Leu Gly Arg Val Gly Ala
   145 150 155 160
   Leu Cys Cys Trp Glu His Leu Ser Pro Leu Ser Lys Tyr Ala Leu Tyr
   165 170 175
   Ser Gln His Glu Ala Ile His Ile Ala Ala Trp Pro Ser Phe Ser Leu
   180 185 190
   Tyr Ser Glu Gln Ala His Ala Leu Ser Ala Lys Val Asn Met Ala Ala
   195 200 205
   Ser Gln Ile Tyr Ser Val Glu Gly Gln Cys Phe Thr Ile Ala Ala Ser
   210 215 220
   Ser Val Val Thr Gln Glu Thr Leu Asp Met Leu Glu Val Gly Glu His
   225 230 235 240
   Asn Ala Pro Leu Leu Lys Val Gly Gly Gly Ser Ser Met Ile Phe Ala
   245 250 255
   Pro Asp Gly Arg Thr Leu Ala Pro Tyr Leu Pro His Asp Ala Glu Gly
   260 265 270
   Leu Ile Ile Ala Asp Leu Asn Met Glu Glu Ile Ala Phe Ala Lys Ala
   275 280 285
   Ile Asn Asp Pro Val Gly His Tyr Ser Lys Pro Glu Ala Thr Arg Leu
   290 295 300
   Val Leu Asp Leu Gly His Arg Asp Pro Met Thr Arg Val His Ser Lys
   305 310 315 320
   Ser Val Thr Arg Glu Glu Ala Pro Glu Gln Gly Val Gln Ser Lys Ile
   325 330 335
   Ala Ser Val Ala Ile Ser His Pro Gln Asp Ser Asp Thr Leu Leu Val
   340 345 350
   Gln Glu Pro Ser
   355
<210> 8
   <211> 1260
   <212> DNA
   <213> Escherichia coli
<220>
   <221> CDS
   <222> (1)..(1257)
   <223> coding for rhaA (L-rhamnose isomerase)
<400> 8
   atg acc act caa ctg gaa cag gcc tgg gag cta gcg aaa cag cgt ttc     48
   Met Thr Thr Gln Leu Glu Gln Ala Trp Glu Leu Ala Lys Gln Arg Phe
    1               5                  10                  15
   gcg gcg gtg ggg att gat gtc gag gag gcg ctg cgc caa ctt gat cgt     96
   Ala Ala Val Gly Ile Asp Val Glu Glu Ala Leu Arg Gln Leu Asp Arg
               20                  25                  30
   tta ccc gtt tca atg cac tgc tgg cag ggc gat gat gtt tcc ggt ttt    144
   Leu Pro Val Ser Met His Cys Trp Gln Gly Asp Asp Val Ser Gly Phe
           35                  40                  45
   gaa aac ccg gaa ggt tcg ctg acc ggg ggg att cag gcc aca ggc aat    192
   Glu Asn Pro Glu Gly Ser Leu Thr Gly Gly Ile Gln Ala Thr Gly Asn
       50                  55                  60
   tat ccg ggc aaa gcg cgt aat gcc agt gag cta cgt gcc gat ctg gaa    240
   Tyr Pro Gly Lys Ala Arg Asn Ala Ser Glu Leu Arg Ala Asp Leu Glu
   65                  70                  75                  80
   cag gct atg cgg ctg att ccg ggg ccg aaa cgg ctt aat tta cat gcc    288
   Gln Ala Met Arg Leu Ile Pro Gly Pro Lys Arg Leu Asn Leu His Ala
                   85                  90                  95
   atc tat ctg gaa tca gat acg cca gtc tcg cgc gac cag atc aaa cca    336
   Ile Tyr Leu Glu Ser Asp Thr Pro Val Ser Arg Asp Gln Ile Lys Pro
               100                 105                 110
   gag cac ttc aaa aac tgg gtt gaa tgg gcg aaa gcc aat cag ctc ggt    384
   Glu His Phe Lys Asn Trp Val Glu Trp Ala Lys Ala Asn Gln Leu Gly
           115                 120                 125
   ctg gat ttt aac ccc tcc tgc ttt tcg cat ccg cta agc gcc gat ggc    432
   Leu Asp Phe Asn Pro Ser Cys Phe Ser His Pro Leu Ser Ala Asp Gly
       130                 135                 140
   ttt acg ctt tcc cat gcc gac gac agc att cgc cag ttc tgg att gat    480
   Phe Thr Leu Ser His Ala Asp Asp Ser Ile Arg Gln Phe Trp Ile Asp
   145                 150                 155                 160
   cac tgc aaa gcc agc cgt cgc gtt tcg gcc tat ttt ggc gag caa ctc    528
   His Cys Lys Ala Ser Arg Arg Val Ser Ala Tyr Phe Gly Glu Gln Leu
                   165                 170                 175
   ggc aca cca tcg gtg atg aac atc tgg atc ccg gat ggt atg aaa gat    576
   Gly Thr Pro Ser Val Met Asn Ile Trp Ile Pro Asp Gly Met Lys Asp
               180                 185                 190
   atc acc gtt gac cgt ctc gcc ccg cgt cag cgt ctg ctg gca gca ctg    624
   Ile Thr Val Asp Arg Leu Ala Pro Arg Gln Arg Leu Leu Ala Ala Leu
           195                 200                 205
   gat gag gtg atc agc gag aag cta aac cct gcg cac cat atc gac gcc     672
   Asp Glu Val Ile Ser Glu Lys Leu Asn Pro Ala His His Ile Asp Ala
       210                 215                 220
   gtt gag agc aaa ttg ttt ggc att ggc gca gag agc tac acg gtt ggc     720
   Val Glu Ser Lys Leu Phe Gly Ile Gly Ala Glu Ser Tyr Thr Val Gly
   225                 230                 235                     240
   tcc aat gag ttt tac atg ggg tat gcc acc agc cgc cag act gcg ctg     768
   Ser Asn Glu Phe Tyr Met Gly Tyr Ala Thr Ser Arg Gln Thr Ala Leu
                   245                 250                 255
   tgc ctg gac gcc ggg cac ttc cac ccg act gaa gtg att tcc gac aag     816
   Cys Leu Asp Ala Gly His Phe His Pro Thr Glu Val Ile Ser Asp Lys
               260                 265                 270
   att tcc gcc gcc atg ctg tat gtg ccg cag ttg ctg ctg cac gtc agc     864
   Ile Ser Ala Ala Met Leu Tyr Val Pro Gln Leu Leu Leu His Val Ser
           275                 280                 285
   cgt ccg gtt cgc tgg gac agc gat cac gta gtg ctg ctg gat gat gaa     912
   Arg Pro Val Arg Trp Asp Ser Asp His Val Val Leu Leu Asp Asp Glu
       290                 295                 300
   acc cag gca att gcc agt gag att gtg cgt cac gat ctg ttt gac cgg     960
   Thr Gln Ala Ile Ala Ser Glu Ile Val Arg His Asp Leu Phe Asp Arg
   305                 310                 315                     320
   gtg cat atc ggc ctt gac ttc ttc gat gcc tct atc aac cgc att gcc     1008
   Val His Ile Gly Leu Asp Phe Phe Asp Ala Ser Ile Asn Arg Ile Ala
                   325                 330                 335
   gcg tgg gtc att ggt aca cgc aat atg aaa aaa gcc ctg ctg cgt gcg     1056
   Ala Trp Val Ile Gly Thr Arg Asn Met Lys Lys Ala Leu Leu Arg Ala
               340                 345                 350
   ttg ctg gaa cct acc gct gac gtg cgc aag ctg gaa gcg gcg ggc gat     1104
   Leu Leu Glu Pro Thr Ala Asp Val Arg Lys Leu Glu Ala Ala Gly Asp
           355                 360                 365
   tac act gcg cgt ctg gca ctg ctg gaa gag cag aaa tcg ttg ccg tgg     1152
   Tyr Thr Ala Arg Leu Ala Leu Leu Glu Glu Gln Lys Ser Leu Pro Trp
       370                 375                 380
   cag gcg gtc tgg gaa atg tat tgc caa cgt cac gat acg cca gca ggt     1200
   Gln Ala Val Trp Glu Met Tyr Cys Gln Arg His Asp Thr Pro Ala Gly
   385                 390                 395                     400
   agc gaa tgg ctg gag agc gtg cgg gct tat gag aaa gaa att ttg agt     1248
   Ser Glu Trp Leu Glu Ser Val Arg Ala Tyr Glu Lys Glu Ile Leu Ser
                   405                 410                 415
   cgc cgc ggg taa                                                     1260
   Arg Arg Gly
<210> 9
   <211> 419
   <212> PRT
   <213> Escherichia coli
<400> 9
   Met Thr Thr Gln Leu Glu Gln Ala Trp Glu Leu Ala Lys Gln Arg Phe
   1               5                   10                  15
   Ala Ala Val Gly Ile Asp Val Glu Glu Ala Leu Arg Gln Leu Asp Arg
               20                  25                  30
   Leu Pro Val Ser Met His Cys Trp Gln Gly Asp Asp Val Ser Gly Phe
           35                  40                  45
   Glu Asn Pro Glu Gly Ser Leu Thr Gly Gly Ile Gln Ala Thr Gly Asn
       50                  55                  60
   Tyr Pro Gly Lys Ala Arg Asn Ala Ser Glu Leu Arg Ala Asp Leu Glu
   65                  70                  75                  80
   Gln Ala Met Arg Leu Ile Pro Gly Pro Lys Arg Leu Asn Leu His Ala
                   85                  90                  95
   Ile Tyr Leu Glu Ser Asp Thr Pro Val Ser Arg Asp Gln Ile Lys Pro
               100                 105                 110
   Glu His Phe Lys Asn Trp Val Glu Trp Ala Lys Ala Asn Gln Leu Gly
           115                 120                 125
   Leu Asp Phe Asn Pro Ser Cys Phe Ser His Pro Leu Ser Ala Asp Gly
       130                 135                 140
   Phe Thr Leu Ser His Ala Asp Asp Ser Ile Arg Gln Phe Trp Ile Asp
   145                 150                 155                 160
   His Cys Lys Ala Ser Arg Arg Val Ser Ala Tyr Phe Gly Glu Gln Leu
                   165                 170                 175
   Gly Thr Pro Ser Val Met Asn Ile Trp Ile Pro Asp Gly Met Lys Asp
               180                 185                 190
   Ile Thr Val Asp Arg Leu Ala Pro Arg Gln Arg Leu Leu Ala Ala Leu
           195                 200                 205
   Asp Glu Val Ile Ser Glu Lys Leu Asn Pro Ala His His Ile Asp Ala
       210                 215                 220
   Val Glu Ser Lys Leu Phe Gly Ile Gly Ala Glu Ser Tyr Thr Val Gly
   225                 230                 235                 240
   Ser Asn Glu Phe Tyr Met Gly Tyr Ala Thr Ser Arg Gln Thr Ala Leu
                   245                 250                 255
   Cys Leu Asp Ala Gly His Phe His Pro Thr Glu Val Ile Ser Asp Lys
               260                 265                 270
   Ile Ser Ala Ala Met Leu Tyr Val Pro Gln Leu Leu Leu His Val Ser
           275                 280                 285
   Arg Pro Val Arg Trp Asp Ser Asp His Val Val Leu Leu Asp Asp Glu
       290                 295                 300
   Thr Gln Ala Ile Ala Ser Glu Ile Val Arg His Asp Leu Phe Asp Arg
   305                 310                 315                 320
   Val His Ile Gly Leu Asp Phe Phe Asp Ala Ser Ile Asn Arg Ile Ala
                   325                 330                 335
   Ala Trp Val Ile Gly Thr Arg Asn Met Lys Lys Ala Leu Leu Arg Ala
               340                 345                 350
   Leu Leu Glu Pro Thr Ala Asp Val Arg Lys Leu Glu Ala Ala Gly Asp
           355                 360                 365
   Tyr Thr Ala Arg Leu Ala Leu Leu Glu Glu Gln Lys Ser Leu Pro Trp
       370                 375                 380
   Gln Ala Val Trp Glu Met Tyr Cys Gln Arg His Asp Thr Pro Ala Gly
   385                 390                 395                 400
   Ser Glu Trp Leu Glu Ser Val Arg Ala Tyr Glu Lys Glu Ile Leu Ser
                   405                 410                 415
   Arg Arg Gly
<210> 10
   <211> 1470
   <212> DNA
   <213> Escherichia coli
<220>
   <221> CDS
   <222> (1)..(1467)
   <223> coding for rhaB (rhamnolukinase)
<400> 10
   atg acc ttt cgc aat tgt gtc gcc gtc gat ctc ggc gca tcc agt ggg    48
   Met Thr Phe Arg Asn Cys Val Ala Val Asp Leu Gly Ala Ser Ser Gly
    1               5                  10                  15
   cgc gtg atg ctg gcg cgt tac gag cgt gaa tgc cgc agc ctg acg ctg    96
   Arg Val Met Leu Ala Arg Tyr Glu Arg Glu Cys Arg Ser Leu Thr Leu
               20                  25                  30
   cgc gaa atc cat cgt ttt aac aat ggg ctg cat agt cag aac ggc tat    144
   Arg Glu Ile His Arg Phe Asn Asn Gly Leu His Ser Gln Asn Gly Tyr
           35                  40                  45
   gtc acc tgg gat gtg gat agc ctt gaa agt gcc att cgc ctt gga tta    192
   Val Thr Trp Asp Val Asp Ser Leu Glu Ser Ala Ile Arg Leu Gly Leu
       50                  55                  60
   aac aag gtg tgc gag gaa ggg att cgt atc gat agc att ggg att gat    240
   Asn Lys Val Cys Glu Glu Gly Ile Arg Ile Asp Ser Ile Gly Ile Asp
   65                  70                  75                  80
   acc tgg ggc gtg gac ttt gtg ctg ctc gac caa cag ggt cag cgt gtg    288
   Thr Trp Gly Val Asp Phe Val Leu Leu Asp Gln Gln Gly Gln Arg Val
                   85                  90                  95
   ggc ctg ccc gtt gct tat cgc gat agc cgc acc aat ggc cta atg gcg    336
   Gly Leu Pro Val Ala Tyr Arg Asp Ser Arg Thr Asn Gly Leu Met Ala
               100                 105                 110
   cag gca caa caa caa ctc ggc aaa cgc gat att tat caa cgt agc ggc    384
   Gln Ala Gln Gln Gln Leu Gly Lys Arg Asp Ile Tyr Gln Arg Ser Gly
           115                 120                 125
   atc cag ttt ctg ccc ttc aat acg ctt tat cag ttg cgt gcg ctg acg    432
   Ile Gln Phe Leu Pro Phe Asn Thr Leu Tyr Gln Leu Arg Ala Leu Thr
       130                 135                 140
   gag caa caa cct gaa ctt att cca cac att gct cac gct ctg ctg atg    480
   Glu Gln Gln Pro Glu Leu Ile Pro His Ile Ala His Ala Leu Leu Met
   145                 150                 155                 160
   ccg gat tac ttc agt tat cgc ctg acc ggc aag atg aac tgg gaa tat    528
   Pro Asp Tyr Phe Ser Tyr Arg Leu Thr Gly Lys Met Asn Trp Glu Tyr
                   165                 170                 175
   acc aac gcc acg acc acg caa ctg gtc aat atc aat agc gac gac tgg    576
   Thr Asn Ala Thr Thr Thr Gln Leu Val Asn Ile Asn Ser Asp Asp Trp
               180                 185                 190
   gac gag tcg cta ctg gcg tgg agc ggg gcc aac aaa gcc tgg ttt ggt    624
   Asp Glu Ser Leu Leu Ala Trp Ser Gly Ala Asn Lys Ala Trp Phe Gly
           195                 200                 205
   cgc ccg acg cat ccg ggt aat gtc ata ggt cac tgg att tgc ccg cag     672
   Arg Pro Thr His Pro Gly Asn Val Ile Gly His Trp Ile Cys Pro Gln
       210                 215                 220
   ggt aat gag att cca gtg gtc gcc gtt gcc agc cat gat acc gcc agc     720
   Gly Asn Glu Ile Pro Val Val Ala Val Ala Ser His Asp Thr Ala Ser
   225                 230                 235                 240
   gcg gtt atc gcc tcg ccg tta aac ggc tca cgt gct gct tat ctc tct     768
   Ala Val Ile Ala Ser Pro Leu Asn Gly Ser Arg Ala Ala Tyr Leu Ser
                   245                 250                 255
   tct ggc acc tgg tca ttg atg ggc ttc gaa agc cag acg cca ttt acc     816
   Ser Gly Thr Trp Ser Leu Met Gly Phe Glu Ser Gln Thr Pro Phe Thr
               260                 265                 270
   aat gac acg gca ctg gca gcc aac atc acc aat gaa ggc ggg gcg gaa     864
   Asn Asp Thr Ala Leu Ala Ala Asn Ile Thr Asn Glu Gly Gly Ala Glu
           275                 280                 285
   ggt cgc tat cgg gtg ctg aaa aat att atg ggc tta tgg ctg ctt cag     912
   Gly Arg Tyr Arg Val Leu Lys Asn Ile Met Gly Leu Trp Leu Leu Gln
       290                 295                 300
   cga gtg ctt cag gag cag caa atc aac gat ctt ccg gcg ctt atc tcc     960
   Arg Val Leu Gln Glu Gln Gln Ile Asn Asp Leu Pro Ala Leu Ile Ser
   305                 310                 315                 320
   gcg aca cag gca ctt ccg gct tgc cgc ttc att atc aat ccc aat gac     1008
   Ala Thr Gln Ala Leu Pro Ala Cys Arg Phe Ile Ile Asn Pro Asn Asp
                   325                 330                 335
   gat cgc ttt att aat cct gag acg atg tgc agc gaa att cag gct gcg     1056
   Asp Arg Phe Ile Asn Pro Glu Thr Met Cys Ser Glu Ile Gln Ala Ala
               340                 345                 350
   tgt cgg gaa acg gcg caa ccg atc ccg gaa agt gat gct gaa ctg gcg     1104
   Cys Arg Glu Thr Ala Gln Pro Ile Pro Glu Ser Asp Ala Glu Leu Ala
           355                 360                 365
   cgc tgc att ttc gac agt ctg gcg ctg ctg tat gcc gat gtg ttg cat     1152
   Arg Cys Ile Phe Asp Ser Leu Ala Leu Leu Tyr Ala Asp Val Leu His
       370                 375                 380
   gag ctg gcg cag ctg cgc ggt gaa gat ttc tcg caa ctg cat att gtc     1200
   Glu Leu Ala Gln Leu Arg Gly Glu Asp Phe Ser Gln Leu His Ile Val
   385                 390                 395                 400
   ggc gga ggc tgc cag aac acg ctg ctc aac cag cta tgc gcc gat gcc     1248
   Gly Gly Gly Cys Gln Asn Thr Leu Leu Asn Gln Leu Cys Ala Asp Ala
                   405                 410                 415
   tgc ggt att cgg gtg atc gcc ggg cct gtt gaa gcc tcg acg ctc ggc     1296
   Cys Gly Ile Arg Val Ile Ala Gly Pro Val Glu Ala Ser Thr Leu Gly
               420                 425                 430
   aat atc ggc atc cag tta atg acg ctg gat gaa ctc aac aat gtg gat     1344
   Asn Ile Gly Ile Gln Leu Met Thr Leu Asp Glu Leu Asn Asn Val Asp
           435                 440                 445
   gat ttc cgt cag gtc gtc agc acc acc gcg aat ctg acc acc ttt acc     1392
   Asp Phe Arg Gln Val Val Ser Thr Thr Ala Asn Leu Thr Thr Phe Thr
       450                 455                 460
   cct aat cct gac agt gaa att gcc cac tat gtg gcg cag att cac tct     1440
   Pro Asn Pro Asp Ser Glu Ile Ala His Tyr Val Ala Gln Ile His Ser
   465                 470                 475                 480
   aca cga cag aca aag gag ctt tgc gca tga                        1470
   Thr Arg Gln Thr Lys Glu Leu Cys Ala
                   485
<210> 11
   <211> 489
   <212> PRT
   <213> Escherichia coli
<400> 11
   Met Thr Phe Arg Asn Cys Val Ala Val Asp Leu Gly Ala Ser Ser Gly
    1               5                  10                  15
   Arg Val Met Leu Ala Arg Tyr Glu Arg Glu Cys Arg Ser Leu Thr Leu
               20                  25                  30
   Arg Glu Ile His Arg Phe Asn Asn Gly Leu His Ser Gln Asn Gly Tyr
           35                  40                  45
   Val Thr Trp Asp Val Asp Ser Leu Glu Ser Ala Ile Arg Leu Gly Leu
       50                  55                  60
   Asn Lys Val Cys Glu Glu Gly Ile Arg Ile Asp Ser Ile Gly Ile Asp
   65                  70                  75                  80
   Thr Trp Gly Val Asp Phe Val Leu Leu Asp Gln Gln Gly Gln Arg Val
                   85                  90                  95
   Gly Leu Pro Val Ala Tyr Arg Asp Ser Arg Thr Asn Gly Leu Met Ala
               100                 105                 110
   Gln Ala Gln Gln Gln Leu Gly Lys Arg Asp Ile Tyr Gln Arg Ser Gly
           115                 120                 125
   Ile Gln Phe Leu Pro Phe Asn Thr Leu Tyr Gln Leu Arg Ala Leu Thr
       130                 135                 140
   Glu Gln Gln Pro Glu Leu Ile Pro His Ile Ala His Ala Leu Leu Met
   145                 150                 155                 160
   Pro Asp Tyr Phe Ser Tyr Arg Leu Thr Gly Lys Met Asn Trp Glu Tyr
                   165                 170                 175
   Thr Asn Ala Thr Thr Thr Gln Leu Val Asn Ile Asn Ser Asp Asp Trp
               180                 185                 190
   Asp Glu Ser Leu Leu Ala Trp Ser Gly Ala Asn Lys Ala Trp Phe Gly
           195                 200                 205
   Arg Pro Thr His Pro Gly Asn Val Ile Gly His Trp Ile Cys Pro Gln
       210                 215                 220
   Gly Asn Glu Ile Pro Val Val Ala Val Ala Ser His Asp Thr Ala Ser
   225                 230                 235                 240
   Ala Val Ile Ala Ser Pro Leu Asn Gly Ser Arg Ala Ala Tyr Leu Ser
                   245                 250                 255
   Ser Gly Thr Trp Ser Leu Met Gly Phe Glu Ser Gln Thr Pro Phe Thr
               260                 265                 270
   Asn Asp Thr Ala Leu Ala Ala Asn Ile Thr Asn Glu Gly Gly Ala Glu
           275                 280                 285
   Gly Arg Tyr Arg Val Leu Lys Asn Ile Met Gly Leu Trp Leu Leu Gln
       290                 295                 300
   Arg Val Leu Gln Glu Gln Gln Ile Asn Asp Leu Pro Ala Leu Ile Ser
   305                 310                 315                 320
   Ala Thr Gln Ala Leu Pro Ala Cys Arg Phe Ile Ile Asn Pro Asn Asp
   325 330 335
   Asp Arg Phe Ile Asn Pro Glu Thr Met Cys Ser Glu Ile Gln Ala Ala
   340 345 350
   Cys Arg Glu Thr Ala Gln Pro Ile Pro Glu Ser Asp Ala Glu Leu Ala
   355 360 365
   Arg Cys Ile Phe Asp Ser Leu Ala Leu Leu Tyr Ala Asp Val Leu His
   370 375 380
   Glu Leu Ala Gln Leu Arg Gly Glu Asp Phe Ser Gln Leu His Ile Val
   385 390 395   400
   Gly Gly Gly Cys Gln Asn Thr Leu Leu Asn Gln Leu Cys Ala Asp Ala
   405 410 415
   Cys Gly Ile Arg Val Ile Ala Gly Pro Val Glu Ala Ser Thr Leu Gly
   420 425 430
   Asn Ile Gly Ile Gln Leu Met Thr Leu Asp Glu Leu Asn Asn Val Asp
   435 440 445
   Asp Phe Arg Gln Val Val Ser Thr Thr Ala Asn Leu Thr Thr Phe Thr
   450 455 460
   Pro Asn Pro Asp Ser Glu Ile Ala His Tyr Val Ala Gln Ile His Ser
   465 470 475 480
   Thr Arg Gln Thr Lys Glu Leu Cys Ala
   485
<210> 12
   <211> 825
   <212> DNA
   <213> Escherichia coli
<220>
   <221> CDS
   <222> (1)..(822)
   <223> coding for rhaD (rhamnulose-phosphate aldolase)
<400> 12
   atg caa aac att act cag tcc tgg ttt gtc cag gga atg atc aaa gcc      48
   Met Gln Asn Ile Thr Gln Ser Trp Phe Val Gln Gly Met Ile Lys Ala
   1               5                   10                  15
   acc acc gac gcc tgg ctg aaa ggc tgg gat gag cgc aac ggc ggc aac      96
   Thr Thr Asp Ala Trp Leu Lys Gly Trp Asp Glu Arg Asn Gly Gly Asn
               20                  25                  30
   ctg acg cta cgc ctg gat gac gcc gat atc gca cca tat cac gac aat      144
   Leu Thr Leu Arg Leu Asp Asp Ala Asp Ile Ala Pro Tyr His Asp Asn
           35                  40                  45
   ttc cac caa caa ccg cgc tat atc ccg ctc agc cag ccc atg cct tta      192
   Phe His Gln Gln Pro Arg Tyr Ile Pro Leu Ser Gln Pro Met Pro Leu
       50                  55                  60
   ctg gca aat aca ccg ttt att gtc acc ggc tcg ggc aaa ttc ttc cgt      240
   Leu Ala Asn Thr Pro Phe Ile Val Thr Gly Ser Gly Lys Phe Phe Arg
   65                  70                  75                  80
   aac gtc cag ctt gat cct gcg gct aac tta ggc atc gta aaa gtc gac      288
   Asn Val Gln Leu Asp Pro Ala Ala Asn Leu Gly Ile Val Lys Val Asp
                   85                  90                  95
   agc gac ggc gcg ggc tac cac att ctt tgg ggg tta acc aac gaa gcc     336
   Ser Asp Gly Ala Gly Tyr His Ile Leu Trp Gly Leu Thr Asn Glu Ala
               100                 105                 110
   gtc ccc act tcc gaa ctt ccg gct cac ttc ctt tcc cac tgc gag cgc     384
   Val Pro Thr Ser Glu Leu Pro Ala His Phe Leu Ser His Cys Glu Arg
           115                 120                 125
   att aaa gcc acc aac ggc aaa gat cgg gtg atc atg cac tgc cac gcc     432
   Ile Lys Ala Thr Asn Gly Lys Asp Arg Val Ile Met His Cys His Ala
       130                 135                 140
   acc aac ctg atc gcc ctc acc tat gta ctt gaa aac gac acc gcg gtc     480
   Thr Asn Leu Ile Ala Leu Thr Tyr Val Leu Glu Asn Asp Thr Ala Val
   145                 150                 155                 160
   ttc act cgc caa ctg tgg gaa ggc agc acc gag tgt ctg gtg gta ttc     528
   Phe Thr Arg Gln Leu Trp Glu Gly Ser Thr Glu Cys Leu Val Val Phe
                   165                 170                 175
   ccg gat ggc gtt ggc att ttg ccg tgg atg gtg ccc ggc acg gac gaa     576
   Pro Asp Gly Val Gly Ile Leu Pro Trp Met Val Pro Gly Thr Asp Glu
               180                 185                 190
   atc ggc cag gcg acc gca caa gag atg caa aaa cat tcg ctg gtg ttg     624
   Ile Gly Gln Ala Thr Ala Gln Glu Met Gln Lys His Ser Leu Val Leu
           195                 200                 205
   tgg ccc ttc cac ggc gtc ttc ggc agc gga ccg acg ctg gat gaa acc     672
   Trp Pro Phe His Gly Val Phe Gly Ser Gly Pro Thr Leu Asp Glu Thr
       210                 215                 220
   ttc ggt tta atc gac acc gca gaa aaa tca gca caa gta tta gtg aag     720
   Phe Gly Leu Ile Asp Thr Ala Glu Lys Ser Ala Gln Val Leu Val Lys
   225                 230                 235                 240
   gtt tat tcg atg ggc ggc atg aaa cag acc atc agc cgt gaa gag ttg     768
   Val Tyr Ser Met Gly Gly Met Lys Gln Thr Ile Ser Arg Glu Glu Leu
                   245                 250                 255
   ata gcg ctc ggc aag cgt ttc ggc gtt acg cca ctc gcc agt gcg ctg     816
   Ile Ala Leu Gly Lys Arg Phe Gly Val Thr Pro Leu Ala Ser Ala Leu
               260                 265                 270
   gcg ctg taa                                                         825
   Ala Leu
<210> 13
   <211> 274
   <212> PRT
   <213> Escherichia coli
<400> 13
   Met Gln Asn Ile Thr Gln Ser Trp Phe Val Gln Gly Met Ile Lys Ala
    1               5                  10                  15
   Thr Thr Asp Ala Trp Leu Lys Gly Trp Asp Glu Arg Asn Gly Gly Asn
               20                  25                  30
   Leu Thr Leu Arg Leu Asp Asp Ala Asp Ile Ala Pro Tyr His Asp Asn
           35                  40                  45
   Phe His Gln Gln Pro Arg Tyr Ile Pro Leu Ser Gln Pro Met Pro Leu
       50                  55                  60
   Leu Ala Asn Thr Pro Phe Ile Val Thr Gly Ser Gly Lys Phe Phe Arg
   65                  70                  75                  80
   Asn Val Gln Leu Asp Pro Ala Ala Asn Leu Gly Ile Val Lys Val Asp
                   85                  90                  95
   Ser Asp Gly Ala Gly Tyr His Ile Leu Trp Gly Leu Thr Asn Glu Ala
               100                 105                 110
   Val Pro Thr Ser Glu Leu Pro Ala His Phe Leu Ser His Cys Glu Arg
           115                 120                 125
   Ile Lys Ala Thr Asn Gly Lys Asp Arg Val Ile Met His Cys His Ala
       130                 135                 140
   Thr Asn Leu Ile Ala Leu Thr Tyr Val Leu Glu Asn Asp Thr Ala Val
   145                 150                 155                 160
   Phe Thr Arg Gln Leu Trp Glu Gly Ser Thr Glu Cys Leu Val Val Phe
                   165                 170                 175
   Pro Asp Gly Val Gly Ile Leu Pro Trp Met Val Pro Gly Thr Asp Glu
               180                 185                 190
   Ile Gly Gln Ala Thr Ala Gln Glu Met Gln Lys His Ser Leu Val Leu
           195                 200                 205
   Trp Pro Phe His Gly Val Phe Gly Ser Gly Pro Thr Leu Asp Glu Thr
       210                 215                 220
   Phe Gly Leu Ile Asp Thr Ala Glu Lys Ser Ala Gln Val Leu Val Lys
   225                 230                 235                 240
   Val Tyr Ser Met Gly Gly Met Lys Gln Thr Ile Ser Arg Glu Glu Leu
                   245                 250                 255
   Ile Ala Leu Gly Lys Arg Phe Gly Val Thr Pro Leu Ala Ser Ala Leu
               260                 265                 270
   Ala Leu
<210> 14
   <211> 939
   <212> DNA
   <213> Escherichia coli
<220>
   <221> CDS
   <222> (1)..(936)
   <223> coding for rhaR (positive regulator for rhaRS operon)
<400> 14
   atg gct ttc tgc aat aac gcg aat ctt ctc aac gta ttt gta cgc cat      48
   Met Ala Phe Cys Asn Asn Ala Asn Leu Leu Asn Val Phe Val Arg His
    1               5                  10                  15
   att gcg aat aat caa ctt cgt tct ctg gcc gag gta gcc acg gtg gcg      96
   Ile Ala Asn Asn Gln Leu Arg Ser Leu Ala Glu Val Ala Thr Val Ala
               20                  25                  30
   cat cag tta aaa ctt ctc aaa gat gat ttt ttt gcc agc gac cag cag     144
   His Gln Leu Lys Leu Leu Lys Asp Asp Phe Phe Ala Ser Asp Gln Gln
           35                  40                  45
   gca gtc gct gtg gct gac cgt tat ccg caa gat gtc ttt gct gaa cat     192
   Ala Val Ala Val Ala Asp Arg Tyr Pro Gln Asp Val Phe Ala Glu His
       50                  55                  60
   aca cat gat ttt tgt gag ctg gtg att gtc tgg cgc ggt aat ggc ctg    240
   Thr His Asp Phe Cys Glu Leu Val Ile Val Trp Arg Gly Asn Gly Leu
    65                 70                  75                  80
   cat gta ctc aac gat cgc cct tat cgc att acc cgt ggc gat ctc ttt    288
   His Val Leu Asn Asp Arg Pro Tyr Arg Ile Thr Arg Gly Asp Leu Phe
                   85                  90                  95
   tac att cat gct gac gat aaa cac tcc tac gct tcc gtt aac gat ctg    336
   Tyr Ile His Ala Asp Asp Lys His Ser Tyr Ala Ser Val Asn Asp Leu
               100                 105                 110
   gtt ttg cag aat att att tat tgc ccg gag cgt ctg aag ctg aat ctt    384
   Val Leu Gln Asn Ile Ile Tyr Cys Pro Glu Arg Leu Lys Leu Asn Leu
           115                 120                 125
   gac tgg cag ggg gcg att ccg gga ttt aac gcc agc gca ggg caa cca    432
   Asp Trp Gln Gly Ala Ile Pro Gly Phe Asn Ala Ser Ala Gly Gln Pro
       130                 135                 140
   cac tgg cgc tta ggt agc atg ggg atg gcg cag gcg cgg cag gtt atc    480
   His Trp Arg Leu Gly Ser Met Gly Met Ala Gln Ala Arg Gln Val Ile
   145                 150                 155                 160
   ggt cag ctt gag cat gaa agt agt cag cat gtg ccg ttt gct aac gaa    528
   Gly Gln Leu Glu His Glu Ser Ser Gln His Val Pro Phe Ala Asn Glu
                   165                 170                 175
   atg gct gag ttg ctg ttc ggg cag ttg gtg atg ttg ctg aat cgc cat    576
   Met Ala Glu Leu Leu Phe Gly Gln Leu Val Met Leu Leu Asn Arg His
               180                 185                 190
   cgt tac acc agt gat tcg ttg ccg cca aca tcc agc gaa acg ttg ctg    624
   Arg Tyr Thr Ser Asp Ser Leu Pro Pro Thr Ser Ser Glu Thr Leu Leu
           195                 200                 205
   gat aag ctg att acc cgg ctg gcg gct agc ctg aaa agt ccc ttt gcg    672
   Asp Lys Leu Ile Thr Arg Leu Ala Ala Ser Leu Lys Ser Pro Phe Ala
       210                 215                 220
   ctg gat aaa ttt tgt gat gag gca tcg tgc agt gag cgc gtt ttg cgt    720
   Leu Asp Lys Phe Cys Asp Glu Ala Ser Cys Ser Glu Arg Val Leu Arg
   225                 230                 235                 240
   cag caa ttt cgc cag cag act gga atg acc atc aat caa tat ctg cga    768
   Gln Gln Phe Arg Gln Gln Thr Gly Met Thr Ile Asn Gln Tyr Leu Arg
                   245                 250                 255
   cag gtc aga gtg tgt cat gcg caa tat ctt ctc cag cat agc cgc ctg    816
   Gln Val Arg Val Cys His Ala Gln Tyr Leu Leu Gln His Ser Arg Leu
               260                 265                 270
   tta atc agt gat att tcg acc gaa tgt ggc ttt gaa gat agt aac tat    864
   Leu Ile Ser Asp Ile Ser Thr Glu Cys Gly Phe Glu Asp Ser Asn Tyr
           275                 280                 285
   ttt tcg gtg gtg ttt acc cgg gaa acc ggg atg acg ccc agc cag tgg    912
   Phe Ser Val Val Phe Thr Arg Glu Thr Gly Met Thr Pro Ser Gln Trp
       290                 295                 300
   cgt cat ctc aat tcg cag aaa gat taa                                939
   Arg His Leu Asn Ser Gln Lys Asp
   305                 310
<210> 15
   <211> 312
   <212> PRT
   <213> Escherichia coli
<400> 15
   Met Ala Phe Cys Asn Asn Ala Asn Leu Leu Asn Val Phe Val Arg His
   1               5                   10                  15
   Ile Ala Asn Asn Gln Leu Arg Ser Leu Ala Glu Val Ala Thr Val Ala
               20                  25                  30
   His Gln Leu Lys Leu Leu Lys Asp Asp Phe Phe Ala Ser Asp Gln Gln
           35                  40                  45
   Ala Val Ala Val Ala Asp Arg Tyr Pro Gln Asp Val Phe Ala Glu His
       50                  55                  60
   Thr His Asp Phe Cys Glu Leu Val Ile Val Trp Arg Gly Asn Gly Leu
   65                  70                  75                  80
   His Val Leu Asn Asp Arg Pro Tyr Arg Ile Thr Arg Gly Asp Leu Phe
                   85                  90                  95
   Tyr Ile His Ala Asp Asp Lys His Ser Tyr Ala Ser Val Asn Asp Leu
               100                 105                 110
   Val Leu Gln Asn Ile Ile Tyr Cys Pro Glu Arg Leu Lys Leu Asn Leu
           115                 120                 125
   Asp Trp Gln Gly Ala Ile Pro Gly Phe Asn Ala Ser Ala Gly Gln Pro
       130                 135                 140
   His Trp Arg Leu Gly Ser Met Gly Met Ala Gln Ala Arg Gln Val Ile
   145                 150                 155                 160
   Gly Gln Leu Glu His Glu Ser Ser Gln His Val Pro Phe Ala Asn Glu
                   165                 170                 175
   Met Ala Glu Leu Leu Phe Gly Gln Leu Val Met Leu Leu Asn Arg His
               180                 185                 190
   Arg Tyr Thr Ser Asp Ser Leu Pro Pro Thr Ser Ser Glu Thr Leu Leu
           195                 200                 205
   Asp Lys Leu Ile Thr Arg Leu Ala Ala Ser Leu Lys Ser Pro Phe Ala
       210                 215                 220
   Leu Asp Lys Phe Cys Asp Glu Ala Ser Cys Ser Glu Arg Val Leu Arg
   225                 230                 235                 240
   Gln Gln Phe Arg Gln Gln Thr Gly Met Thr Ile Asn Gln Tyr Leu Arg
                   245                 250                 255
   Gln Val Arg Val Cys His Ala Gln Tyr Leu Leu Gln His Ser Arg Leu
               260                 265                 270
   Leu Ile Ser Asp Ile Ser Thr Glu Cys Gly Phe Glu Asp Ser Asn Tyr
           275                 280                 285
   Phe Ser Val Val Phe Thr Arg Glu Thr Gly Met Thr Pro Ser Gln Trp
       290                 295                 300
   Arg His Leu Asn Ser Gln Lys Asp
   305                 310
<210> 16
   <211> 837
   <212> DNA
   <213> Escherichia coli
<220>
   <221> CDS
   <222> (1)..(834)
   <223> coding for rhaS (positive regulator of rhaBAD operon)
<400> 16
   atg acc gta tta cat agt gtg gat ttt ttt ccg tct ggt aac gcg tcc     48
   Met Thr Val Leu His Ser Val Asp Phe Phe Pro Ser Gly Asn Ala Ser
    1               5                  10                  15
   gtg gcg ata gaa ccc cgg ctc ccg cag gcg gat ttt cct gaa cat cat     96
   Val Ala Ile Glu Pro Arg Leu Pro Gln Ala Asp Phe Pro Glu His His
               20                  25                  30
   cat gat ttt cat gaa att gtg att gtc gaa cat ggc acg ggt att cat    144
   His Asp Phe His Glu Ile Val Ile Val Glu His Gly Thr Gly Ile His
           35                  40                  45
   gtg ttt aat ggg cag ccc tat acc atc acc ggt ggc acg gtc tgt ttc    192
   Val Phe Asn Gly Gln Pro Tyr Thr Ile Thr Gly Gly Thr Val Cys Phe
       50                  55                  60
   gta cgc gat cat gat cgg cat ctg tat gaa cat acc gat aat ctg tgt    240
   Val Arg Asp His Asp Arg His Leu Tyr Glu His Thr Asp Asn Leu Cys
   65                  70                  75                  80
   ctg acc aat gtg ctg tat cgc tcg ccg gat cga ttt cag ttt ctc gcc    288
   Leu Thr Asn Val Leu Tyr Arg Ser Pro Asp Arg Phe Gln Phe Leu Ala
                   85                  90                  95
   ggg ctg aat cag ttg ctg cca caa gag ctg gat ggg cag tat ccg tct    336
   Gly Leu Asn Gln Leu Leu Pro Gln Glu Leu Asp Gly Gln Tyr Pro Ser
               100                 105                 110
   cac tgg cgc gtt aac cac agc gta ttg cag cag gtg cga cag ctg gtt    384
   His Trp Arg Val Asn His Ser Val Leu Gln Gln Val Arg Gln Leu Val
           115                 120                 125
   gca cag atg gaa cag cag gaa ggg gaa aat gat tta ccc tcg acc gcc    432
   Ala Gln Met Glu Gln Gln Glu Gly Glu Asn Asp Leu Pro Ser Thr Ala
       130                 135                 140
   agt cgc gag atc ttg ttt atg caa tta ctg ctc ttg ctg cgt aaa agc    480
   Ser Arg Glu Ile Leu Phe Met Gln Leu Leu Leu Leu Leu Arg Lys Ser
   145                 150                 155                 160
   agt ttg cag gag aac ctg gaa aac agc gca tca cgt ctc aac ttg ctt    528
   Ser Leu Gln Glu Asn Leu Glu Asn Ser Ala Ser Arg Leu Asn Leu Leu
                   165                 170                 175
   ctg gcc tgg ctg gag gac cat ttt gcc gat gag gtg aat tgg gat gcc    576
   Leu Ala Trp Leu Glu Asp His Phe Ala Asp Glu Val Asn Trp Asp Ala
               180                 185                 190
   gtg gcg gat caa ttt tct ctt tca ctg cgt acg cta cat cgg cag ctt    624
   Val Ala Asp Gln Phe Ser Leu Ser Leu Arg Thr Leu His Arg Gln Leu
           195                 200                 205
   aag cag caa acg gga ctg acg cct cag cga tac ctg aac cgc ctg cga    672
   Lys Gln Gln Thr Gly Leu Thr Pro Gln Arg Tyr Leu Asn Arg Leu Arg
       210                 215                 220
   ctg atg aaa gcc cga cat ctg cta cgc cac agc gag gcc agc gtt act    720
   Leu Met Lys Ala Arg His Leu Leu Arg His Ser Glu Ala Ser Val Thr
   225                 230                 235                 240
   gac atc gcc tat cgc tgt gga ttc agc gac agt aac cac ttt tcg acg    768
   Asp Ile Ala Tyr Arg Cys Gly Phe Ser Asp Ser Asn His Phe Ser Thr
                   245                     250                 255
   ctt ttt cgc cga gag ttt aac tgg tca ccg cgt gat att cgc cag gga    816
   Leu Phe Arg Arg Glu Phe Asn Trp Ser Pro Arg Asp Ile Arg Gln Gly
               260                     265                 270
   cgg gat ggc ttt ctg caa taa                                        837
   Arg Asp Gly Phe Leu Gln
           275
<210> 17
   <211> 278
   <212> PRT
   <213> Escherichia coli
<400> 17
   Met Thr Val Leu His Ser Val Asp Phe Phe Pro Ser Gly Asn Ala Ser
    1               5                  10                  15
   Val Ala Ile Glu Pro Arg Leu Pro Gln Ala Asp Phe Pro Glu His His
               20                  25                  30
   His Asp Phe His Glu Ile Val Ile Val Glu His Gly Thr Gly Ile His
           35                  40                  45
   Val Phe Asn Gly Gln Pro Tyr Thr Ile Thr Gly Gly Thr Val Cys Phe
       50                  55                  60
   Val Arg Asp His Asp Arg His Leu Tyr Glu His Thr Asp Asn Leu Cys
   65                  70                  75                  80
   Leu Thr Asn Val Leu Tyr Arg Ser Pro Asp Arg Phe Gln Phe Leu Ala
                   85                  90                  95
   Gly Leu Asn Gln Leu Leu Pro Gln Glu Leu Asp Gly Gln Tyr Pro Ser
               100                 105                 110
   His Trp Arg Val Asn His Ser Val Leu Gln Gln Val Arg Gln Leu Val
           115                 120                 125
   Ala Gln Met Glu Gln Gln Glu Gly Glu Asn Asp Leu Pro Ser Thr Ala
       130                 135                 140
   Ser Arg Glu Ile Leu Phe Met Gln Leu Leu Leu Leu Leu Arg Lys Ser
   145                 150                 155                 160
   Ser Leu Gln Glu Asn Leu Glu Asn Ser Ala Ser Arg Leu Asn Leu Leu
                   165                 170                 175
   Leu Ala Trp Leu Glu Asp His Phe Ala Asp Glu Val Asn Trp Asp Ala
               180                 185                 190
   Val Ala Asp Gln Phe Ser Leu Ser Leu Arg Thr Leu His Arg Gln Leu
           195                 200                 205
   Lys Gln Gln Thr Gly Leu Thr Pro Gln Arg Tyr Leu Asn Arg Leu Arg
       210                 215                 220
   Leu Met Lys Ala Arg His Leu Leu Arg His Ser Glu Ala Ser Val Thr
   225                 230                 235                 240
   Asp Ile Ala Tyr Arg Cys Gly Phe Ser Asp Ser Asn His Phe Ser Thr
                   245                 250                 255
   Leu Phe Arg Arg Glu Phe Asn Trp Ser Pro Arg Asp Ile Arg Gln Gly
               260                 265                 270
   Arg Asp Gly Phe Leu Gln
           275
<210> 18
   <211> 1035
   <212> DNA
   <213> Escherichia coli
<220>
   <221> CDS
   <222> (1)..(1032)
   <223> coding for rhaT (rhamnose transport protein)
<400> 18
   atg agt aac gcg att acg atg ggg ata ttt tgg cat ttg atc ggc gcg      48
   Met Ser Asn Ala Ile Thr Met Gly Ile Phe Trp His Leu Ile Gly Ala
    1               5                  10                  15
   gcc agt gca gcc tgt ttt tac gct ccg ttc aaa aaa gta aaa aaa tgg      96
   Ala Ser Ala Ala Cys Phe Tyr Ala Pro Phe Lys Lys Val Lys Lys Trp
               20                  25                  30
   tca tgg gaa acc atg tgg tca gtc ggt ggg att gtt tcg tgg att att     144
   Ser Trp Glu Thr Met Trp Ser Val Gly Gly Ile Val Ser Trp Ile Ile
           35                  40                  45
   ctg ccg tgg gcc atc agc gcc ctg tta cta ccg aat ttc tgg gcg tat     192
   Leu Pro Trp Ala Ile Ser Ala Leu Leu Leu Pro Asn Phe Trp Ala Tyr
       50                  55                  60
   tac agc tcg ttt agt ctc tct acg cga ctg cct gtt ttt ctg ttc ggc     240
   Tyr Ser Ser Phe Ser Leu Ser Thr Arg Leu Pro Val Phe Leu Phe Gly
   65                  70                  75                  80
   gct atg tgg ggg atc ggt aat atc aac tac ggc ctg acc atg cgt tat     288
   Ala Met Trp Gly Ile Gly Asn Ile Asn Tyr Gly Leu Thr Met Arg Tyr
                   85                  90                  95
   ctc ggc atg tcg atg gga att ggc atc gcc att ggc att acg ttg att     336
   Leu Gly Met Ser Met Gly Ile Gly Ile Ala Ile Gly Ile Thr Leu Ile
               100                 105                 110
   gtc ggt acg ctg atg acg cca att atc aac ggc aat ttc gat gtg ttg     384
   Val Gly Thr Leu Met Thr Pro Ile Ile Asn Gly Asn Phe Asp Val Leu
           115                 120                 125
   att agc acc gaa ggc gga cgc atg acg ttg ctc ggc gtt ctg gtg gcg     432
   Ile Ser Thr Glu Gly Gly Arg Met Thr Leu Leu Gly Val Leu Val Ala
       130                 135                 140
   ctg att ggc gta ggg att gta act cgc gcc ggg cag ttg aaa gag cgc     480
   Leu Ile Gly Val Gly Ile Val Thr Arg Ala Gly Gln Leu Lys Glu Arg
   145                 150                 155                 160
   aag atg ggc att aaa gcc gaa gag ttc aat ctg aaa aaa ggg ctg gtg     528
   Lys Met Gly Ile Lys Ala Glu Glu Phe Asn Leu Lys Lys Gly Leu Val
                   165                 170                 175
   ctg gcg gtg atg tgc ggc att ttc tct gcc ggg atg tcc ttt gcg atg     576
   Leu Ala Val Met Cys Gly Ile Phe Ser Ala Gly Met Ser Phe Ala Met
               180                 185                 190
   aac gcc gca aaa ccg atg cat gaa gcc gct gcc gca ctt ggc gtc gat     624
   Asn Ala Ala Lys Pro Met His Glu Ala Ala Ala Ala Leu Gly Val Asp
           195                 200                 205
   cca ctg tat gtc gct ctg cca agc tat gtt gtc atc atg ggc ggc ggc      672
   Pro Leu Tyr Val Ala Leu Pro Ser Tyr Val Val Ile Met Gly Gly Gly
       210                 215                 220
   gcg atc att aac ctc ggt ttc tgt ttt att cgt ctg gca aaa gtg aag      720
   Ala Ile Ile Asn Leu Gly Phe Cys Phe Ile Arg Leu Ala Lys Val Lys
   225                 230                 235                 240
   gat ttg tcg cta aaa gcc gac ttc tcg ctg gca aaa tcg ctg atc att      768
   Asp Leu Ser Leu Lys Ala Asp Phe Ser Leu Ala Lys Ser Leu Ile Ile
                   245                 250                 255
   cac aat gtg tta ctc tcg aca ctg ggc ggg ttg atg tgg tat ctg caa      816
   His Asn Val Leu Leu Ser Thr Leu Gly Gly Leu Met Trp Tyr Leu Gln
               260                 265                 270
   ttc ttt ttc tat gcc tgg ggc cac gcc cgc att ccg gcg cag tat gac      864
   Phe Phe Phe Tyr Ala Trp Gly His Ala Arg Ile Pro Ala Gln Tyr Asp
           275                 280                 285
   tac atc agt tgg atg ctg cat atg agt ttc tat gta ttg tgc ggc ggt      912
   Tyr Ile Ser Trp Met Leu His Met Ser Phe Tyr Val Leu Cys Gly Gly
       290                 295                 300
   atc gtc ggg ctg gtg ctg aaa gag tgg aac aat gca gga cgc cgt ccg      960
   Ile Val Gly Leu Val Leu Lys Glu Trp Asn Asn Ala Gly Arg Arg Pro
   305                 310                 315                 320
   gta acg gtg ttg agc ctc ggt tgt gtg gtg att att gtc gcc gct aac      1008
   Val Thr Val Leu Ser Leu Gly Cys Val Val Ile Ile Val Ala Ala Asn
                   325                 330                 335
   atc gtc ggc atc ggc atg gcg aat taa                                  1035
   Ile Val Gly Ile Gly Met Ala Asn
               340
<210> 19
   <211> 344
   <212> PRT
   <213> Escherichia coli
<400> 19
   Met Ser Asn Ala Ile Thr Met Gly Ile Phe Trp His Leu Ile Gly Ala
    1               5                  10                  15
   Ala Ser Ala Ala Cys Phe Tyr Ala Pro Phe Lys Lys Val Lys Lys Trp
               20                  25                  30
   Ser Trp Glu Thr Met Trp Ser Val Gly Gly Ile Val Ser Trp Ile Ile
           35                  40                  45
   Leu Pro Trp Ala Ile Ser Ala Leu Leu Leu Pro Asn Phe Trp Ala Tyr
       50                  55                  60
   Tyr Ser Ser Phe Ser Leu Ser Thr Arg Leu Pro Val Phe Leu Phe Gly
   65                  70                  75                  80
   Ala Met Trp Gly Ile Gly Asn Ile Asn Tyr Gly Leu Thr Met Arg Tyr
                   85                  90                  95
   Leu Gly Met Ser Met Gly Ile Gly Ile Ala Ile Gly Ile Thr Leu Ile
               100                 105                 110
   Val Gly Thr Leu Met Thr Pro Ile Ile Asn Gly Asn Phe Asp Val Leu
           115                 120                 125
   Ile Ser Thr Glu Gly Gly Arg Met Thr Leu Leu Gly Val Leu Val Ala
       130                 135                 140
   Leu Ile Gly Val Gly Ile Val Thr Arg Ala Gly Gln Leu Lys Glu Arg
   145 150 155 160
   Lys Met Gly Ile Lys Ala Glu Glu Phe Asn Leu Lys Lys Gly Leu Val
   165 170 175
   Leu Ala Val Met Cys Gly Ile Phe Ser Ala Gly Met Ser Phe Ala Met
   180 185 190
   Asn Ala Ala Lys Pro Met His Glu Ala Ala Ala Ala Leu Gly Val Asp
   195 200 205
   Pro Leu Tyr Val Ala Leu Pro Ser Tyr Val Val Ile Met Gly Gly Gly
   210 215 220
   Ala Ile Ile Asn Leu Gly Phe Cys Phe Ile Arg Leu Ala Lys Val Lys
   225 230 235 240
   Asp Leu Ser Leu Lys Ala Asp Phe Ser Leu Ala Lys Ser Leu Ile Ile
   245 250 255
   His Asn Val Leu Leu Ser Thr Leu Gly Gly Leu Met Trp Tyr Leu Gln
   260 265 270
   Phe Phe Phe Tyr Ala Trp Gly His Ala Arg Ile Pro Ala Gln Tyr Asp
   275 280 285
   Tyr Ile Ser Trp Met Leu His Met Ser Phe Tyr Val Leu Cys Gly Gly
   290 295 300
   Ile Val Gly Leu Val Leu Lys Glu Trp Asn Asn Ala Gly Arg Arg Pro
   305 310 315 320
   Val Thr Val Leu Ser Leu Gly Cys Val Val Ile Ile Val Ala Ala Asn
   325 330 335
   Ile Val Gly Ile Gly Met Ala Asn
   340

## Beschreibung

Die vorliegende Erfindung betrifft Verfahren zur Expression von Nukleinsäuresequenzen in prokaryontischen Wirtszellen, wobei man mindestens ein in besagten Wirtszellen episomal replizierbares DNA-Konstrukt umfassend eine zu exprimierende Nukleinsäuresequenz unter transkriptioneller Kontrolle eines L-Rhamnose-induzierbaren Promotors, wobei besagter Promotor in Bezug auf besagte Nukleinsäuresequenz heterolog ist, in besagte Wirtszellen einbringt und die Expression besagter Nukleinsäuresequenz durch Zugabe von L-Rhamnose induziert, dadurch gekennzeichnet, dass die prokaryontische Wirtszelle zumindest defizient ist in Bezug auf eine L-Rhamnose-Isomerase. Die Erfindung betrifft ferner prokaryontische Wirtszellen, die zumindest defizient sind in Bezug auf eine L-Rhamnose-Isomerase und mindestens ein in besagter Wirtzelle replizierbares DNA-Konstrukt enthalten, welches eine zu exprimierende Nukleinsäuresequenz unter transkriptioneller Kontrolle eines durch L-Rhamnose-induzierbaren Promotors umfaßt, wobei besagter Promotor in Bezug auf besagte Nukleinsäuresequenz heterolog ist.

Die heterologe Expression von Genen ist eine ökonomische Möglichkeit, Enzyme und andere Proteine für pharmazeutische und industrielle Verwendungszwecke herzustellen. Besagte Expressionen werden nach wie vor überwiegend mit Stämmen von Escherichia coli realisiert. Zur Herstellung rekombinanter Proteine sind eine Vielzahl von Systemen bekannt, die sich unterschiedlicher Wirtsorganismen und Genexpressionskassetten bedienen. Obgleich zahlreiche Systeme und Verfahren zur Expression rekombinanter Proteine in mikrobiellen Systemen beschrieben sind, basieren die Expressionssysteme für gram-negative Bakterien wie Escherichia coli auf einem sehr limitierten Repertoir bakterieller Promotoren. Am verbreitesten sind der Laktose-Promotor [lac] (Yanisch-Perron et al. (1985) Gene 33: 103-109) und der Tryptophan-Promotor [trp] (Goeddel et al. (1980) Nature (London) 287: 411-416) sowie Hybridpromotoren der vorgenannten [lac und trc] (Brosius (1984) Gene 27:161-172; Amanna & Brosius (1985) Gene 40: 183-190). Weitere Beispiele sind die Promotoren PL und PR des λ-Phagen (Elvin et al. (1990) Gene 37:123-126), der Phage T7-Promotor (Tabor & Richardson (1998) Proc Natl Acad Sci USA 82:1074-1078) und der Promotor der alkalischen Phosphatase [pho] (Chang et al. (1986) Gene 44:121-125).

Mit der heterologen Expression sind verschiedene Probleme wie beispielsweise die Toxizität des Genproduktes, zu geringe Expressionsraten oder Bildung von unlöslichen Proteinaggregaten ("inclusion bodies") verbunden. Viele der oben beschriebenen Promotoren sind für Anwendungen ungeeignet, bei denen das zu exprimierende rekombinante Proteine eine toxische Wirkung auf den jeweiligen Wirt hat. In diesen Fällen ist eine möglichst strikte Regulation der Expression wünschenswert. Dazu können sogenannte induzierbare Promotorsysteme eingesetzt werden, die mittels Zugabe eines Induktors oder eines anderen exogenen Stimulus (z.B. Hitze) induziert werden können. Besagte induzierbare Promotorsysteme bestehen in der Regel aus einer Promotor/Regulator-Kombination, wobei der Regulator beispielsweise ein Protein darstellt, welches in Kombination mit einem exogenen Stimulus die Transkription ausgehend von dem entsprechenden Promotor induziert. Beispielhaft zu nennen ist die Kombination eines Promotors mit einem Repressor wie z.B. dem lac-Repressor (Studier FW et al. (1990) Methods in Enzymol 185:60-89; Dubendorff JW & Studier FW (1991) J Mol Biol 219:45- 59). Die reprimierende Wirkung dieses Repressor kann durch Zugabe eines natürlichen Induktors (z.B. Laktose) oder eines künstlichen Induktors (z.B. Isopropyl-β-D-thiogalactopyranosid; IPTG) aufgehoben und die Expression so initiiert werden.
IPTG kann im Gegensatz zu Laktose nicht verstoffwechselt werden und gewährt so eine langanhaltende Induktion. Ein weiteres Beispiele für diese induzierbaren Promotoren ist der durch Arabinose induzierbare araB Promotor (US 5,028,530; Guzman LM et al. (1995) J Bacteriol 177:4121-4130).

IPTG und andere synthetische Induktoren sind sehr teuer und wirken sich teilweise nachteilig auf das Wachstum der Organismen aus, was eine Anwendung im großindustriellen Maßstab unrentabel macht.

Physiologische Induktoren wie Aminosäuren (z.B. Tryptophan) und Zucker (Arabinose) sind in der Regel zwar billiger, werden aber vom Organismus verstoffwechselt, so dass sie in einer Zellanzucht, insbesondere bei Hochdichtezellfermentationen, in erheblichen Mengen hinzugefügt und/oder nachträglich zudosiert werden müssen. Außerdem können Metaboliten dieser Verbindungen auch schädlich für die weitere Anzucht sein, z.B. bei der Entstehung von Acetat aus Zuckern.

WO 01/73082 beschreibt ein Verfahren zur Expression rekombinanter Proteine unter Kontrolle des induzierbaren araB Promotors in einem E.coli Wirtsorganismus, der eine Defizienz für den aktiven Transport des Induktors Arabinose aufweist. Als Vorteil wird hier geltend gemacht, dass kein aktiver Transport sondern lediglich passiver Transport (durch Diffusion) stattfinden kann. Dadurch kann die intrazellulare Arabinose-Konzentration und somit auch die Expressionsinduktion besser kontrolliert werden. In einigen der aufgeführten Beispiele wird ein E.coli Stamm (E104) eingesetzt, der eine Defizienz in den Arabinose-metaboliserenden Enzymen Ribulokinase (AraB) und L-Ribulose-5-phosphat-4-Epimerase (AraD) aufweist. Gemäß den Expressionsdaten hat diese Defizienz jedoch keine wesentliche Auswirkung auf die Expressionshöhen. Das Arabinose-induzierbare System hat verschiedene Nachteile:
a) Arabinose hat bereits ab Konzentrationen von größer 0,1 mM einen wachstumshemmenden Effekt auf die Bakterienkultur, der auch mit dem in WO 01/73082 beschriebenen Verfahren nur bedingt kompensiert werden kann (vgl. Tabelle 4, WO 01/73082).
b) Der Arabinose-induzierbare Promotor ist in Abwesenheit von Arabinose nicht gänzlich inaktiv, sondern besitzt eine recht hohe Basisaktivität (vgl. Tabelle 5, WO 01/73082).
c) Die Qualität der exprimierten rekombinanten Proteine ist abhängig von der Zelldichte und nimmt mit zunehmenden Zelldichten ab (De Lisa MP et al. (1999) Biotechnol Bioeng 65:54-64).

Beschrieben ist der Escherichia coli Stamm JB1204 (CGSC6999, Bulawa & Raetz (1984) J Biol Chem 259:11257-11264), der die Transposoninsertion "rha-14::Tn10" aufweist, wobei zur Sequenz oder Funktion von "rha-14" keine genaueren Angaben gemacht werden.

Die Ausnahme und Verstoffwechslung von L-Rhamnose in Bakterien wie E.coli ist beschrieben. L-Rhamnose wird über ein aktives Transportsystem (RhaT) in die Zellen aufgenommen, mit einer Isomerase (RhaA) in L-Rhamnulose überführt, die dann weiter durch die Rhamnulose-1-Phosphatase (RhaB) phosphoryliert und durch eine Aldolase (RhaD) zu Dihydroxyacetonphosphat und Lactaldehyd hydrolysiert wird. Die Gene rhaBAD bilden ein Operon und werden mit Hilfe des sogenannten rhaP_{BAD}-Promotors transkribiert. Das Rhamnosesystems zeichnet sich gegenüber anderen Systemen dadurch aus, dass zwei Aktivatoren RhaS und RhaR zur Regulation erforderlich sind. Beide bilden eine Transkriptionseinheit und werden entgegengesetzt zu rhaBAD transkribiert. In Anwesentheit von L-Rhamnose bindet RhaR an den rhaP_{RS}-Promotor und initiiert seine eigene Expression als auch die RhaS-Expression. RhaS wiederum bindet nach Aktivierung durch L-Rhamnose als Effektor an den rhaP_{BAD}-Promotor und den separaten rhaP_{T}-Promotor des rhaT-Gens und aktiviert die Transkription der Strukturgene (Moralejo P et al. (1993) J Bacteriol175:5585-5594; Tobin JF et al. (1990) J Mol Biol 211:1-4; Chen YM et al. (1987) J Bacteriol 169:3712-3719; Egan SM et al. (1993) J Mol Biol 243:87-98). Die Kombination zweier Aktivatoren bedingt eine ungewöhnlich strikte Expressionskontrolle durch den rhaP_{BAD}-Promotor. Ein Vergleich des Arabinose-induzierbaren araB-Promotors und des Rhamnose-induzierbaren rhaP_{BAD}-Promotor zeigt, dass letzterer wesentlich strikter reguliert ist und in Abwesentheit des Induktors Rhamnose quasi einen Null-Phänotyp repräsentiert (Haldimann A et al. (1998) J Bacteriol 180(5):1277-1286).

WO 01/32890 beschreibt die Herstellung von L-Pantolacton-Hydrolase mit Escherichia coli TG1 pDHE681 bzw. Derivaten, wobei L-Rhamnose als Induktor für die Genexpression des Enzyms eingesetzt wird. Da L-Rhamnose von E. coli gut verstoffwechselt wird, muß die umgesetzte L-Rhamnose durch Zufütterung nachgeführt werden. Dies bedeutet einen erheblichen experimentellen Aufwand und erhöht die Kosten für das Anzuchtmedium.

Beschrieben sind ferner Expressionssysteme zur Fermentation unter hohen Zelldichten unter Verwendung des L-Rhamnose-induzierbaren rhaBAD-Promotors und eines E.coli Stamm, der eine gezielt-eingeführte Defizienz in der L-Rhamnulosekinase (rhaB) aufweist (Stumpp T et al. (2000) Biospectrum 6(1):33-36; Wilms B et al. (2001) Biotechnol Bioeng 73(2): 95-103). RhaB wurde hier bewußt ausgewählt, da es der erste irreversible Schritt der Metabolisierung von L-Rhamnose ist (vgl. Wilms B et al. (2001) Biotechnol Bioeng 73(2) S.98, linke Spalte, Z.4-8). Eine optimale Induktion kann in diesen System mit L-Rhamnose-Konzentrationen von 2 g/L erreicht werden (vgl. Wilms B et al. (2001) Biotechnol Bioeng 73(2) S.102, linke Spalte, 2. Absatz Z.1-4). Diese Konzentrationen sind immer noch sehr hoch. Bei einem durchschnittlichen Preis von ca. 100 €/kg L-Rhamnose würden bei einem 10 m³ Fermenter noch Kosten von 2000 € nur für die L-Rhamnose anfallen.

Beschrieben sind ferner eng-regulierte Rhamnose-induzierbare Expressionsysteme, bei denen mittels homologer Rekombination das hinter dem endogenen rhaP_{BAD}-Promotor lokalisierte Rhamnose-Operon (BAD) gegen das PhoB-Gen (Transkriptionsaktivator) ausgetauscht wurde (Haldimann A et al. (1998) J Bacteriol 180(5) :1277-1286). Das hier beschriebene System ist zwar gut geeignet, um Regulatorstudien zu betreiben, da eine sehr enge Regulation gewährleistet ist. Es ist jedoch zur Überexpression - insbesondere unter hochdichten Zellkulturbedingungen - wenig geeignet, da - aufgrund des Austausches des chromosomalen Rhamnose-Operons - jeweils nur eine Kopie der rhaP_{BAD}-Promotor gesteuerten Expressionskassette eingebracht werden kann. Ferner ist der Austausch von Genen mittels homologer Rekombination aufwendig und erfordert eine mühsame Selektion und Charakterisierung entsprechend modifizierter Organismen. Dies macht das beschriebene Verfahren untauglich für den Routineeinsatz.

Es stellte sich die Aufgabe, ein verbessertes Verfahren für die Expression von Nukleinsäuren - und bevorzugt rekombinanten Proteinen - bereitzustellen, was mit geringen L-Rhamnose-Mengen hohe Expressionspiegel ergibt. Diese Aufgabe wird durch die vorliegende Erfindung gelöst.

Ein erster Gegenstand der Erfindung betrifft Verfahren zur Expression von Nukleinsäuresequenzen in prokaryontischen Wirtszellen, wobei man
a) mindestens ein in besagten Wirtszellen episomal replizierbares, DNA-Konstrukt umfassend eine zu exprimierende Nukleinsäuresequenz unter transkriptioneller Kontrolle eines L-Rhamnose-induzierbaren Promotors, wobei besagter Promotor in Bezug auf besagte Nukleinsäuresequenz heterolog ist, in besagte Wirtzellen einbringt und
b) prokaryontischen Wirtszellen selektioniert, welche besagtes DNA-Konstrukt in episomaler Form enthalten und
c) die Expression besagter Nukleinsäuresequenz durch Zugabe von L-Rhamnose zu einer Kultur besagter selektionierter Wirtzellen induziert,
dadurch gekennzeichnet, dass die prokaryontische Wirtszelle zumindest defizient ist in Bezug auf L-Rhamnose-Isomerase.

In einer bevorzugten Ausführungsform bedingt die Expression der zu exprimierenden Nukleinsäuresequenz die Produktion eines durch besagte Nukleinsäuresequenz kodierten Proteins, so dass das erfindungsgemäße Verfahren zur Herstellung rekombinanter Proteine eingesetzt werden kann.

In einer weiterhin bevorzugten Ausführungsform kann eine zusätzliche Defizienz in einem oder mehreren weiteren L-Rhamnose-metabolisierenden bzw. transportierenden Proteinen vorliegen.

Ein weiterer Gegenstand der Erfindung betrifft eine prokaryontische Wirtszelle, die zumindest defizient ist in Bezug auf L-Rhamnose-Isomerase und mindestens ein in besagter Wirtzelle replizierbares DNA-Konstrukt enthält, welches eine zu exprimierende Nukleinsäuresequenz unter transkriptioneller Kontrolle eines durch L-Rhamnose-induzierbaren Promotors umfaßt, wobei besagter Promotor in Bezug auf besagte Nukleinsäuresequenz heterolog ist.

In einer bevorzugten Ausführungsform kann die erfindungsgemäße prokaryontische Wirtszelle eine zusätzliche Defizienz in einem oder mehreren weiteren L-Rhamnose-metabolisierenden bzw. transportierenden Proteinen vorliegen.

Ausserdem betrifft die Erfindung ein Verfahren zur Herstellung von Herstellung von rekombinanten Proteinen, Enzymen und anderen Feinchemikalien wie beispielweise chiraler Carbonsäuren unter Einsatz einer der erfindungsgemäßen prokaryontischen Wirtszellen oder einer Präparationen derselben.

Das erfindunsggemäße Verfahren hat verschiedene Vorteile:
1. Es ist einfach anzuwenden, da ausgehend von einem Wirtsstamm durch einfache Transformation der entsprechende Expressionsstamm generiert werden kann, ohne dass eine Insertion mittels homologer Rekombination in das Genom (wie bei Haldimann A et al. (1998) J Bacteriol 180(5):1277-1286) und eine aufwendige Selektion korrekt modifizierter Organismen erforderlich wäre.
2. Die im Rahmen der Erfindung zur Verfügung gestellten Expressionskassetten und -vektoren sind leicht handhabbar. Der beispielhaft eingesetzte rhaP_{BAD}-Promotor hat eine Länge von lediglich 123 Basenpaaren.
3. Da L-Rhamnose, insbesondere bei C-Quellen-limitierten Fermentationen, von E.coli verstoffwechselt wird, entsteht bei Standardverfahren ein hoher Verbrauch von L-Rhamnose (Zufütterung) und damit hohe Mediumskosten. Durch den niedrigen L-Rhamnose-Bedarf des erfindungsgemäßen Verfahrens (<1 % im Vergleich zu L-Rhamnose-metabolisierenden Stämmen) werden die Kosten für das Fermentationsmedium und damit die Biokatalysator-Herstellung erheblich gesenkt. Durch die Bereitstellung des erfindungsgemäßen Verfahrens, ist die Herstellung rekombinanter Proteine (z.B. Nitrilase, L-Pantolacton-Hydrolase) durch Hochdichtezell-Fermentation (z.B. der bereitgestellten E.coli-TG10-Stämme) ohne laufende Rhamnose-Zufütterung möglich.
4. Die Regulation des beschriebenen Systems erwies sich als außergewöhnlich dicht und ergab bereits bei sehr geringen Konzentrationen des Induktors L-Rhamnose von bis zu 0,05 g/l nach wie vor die maximale Induktion, während bei Abwesenheit des Induktors keinerlei Promotoraktivität detektiert werden konnte. Damit eignet sich das System auch hervorragend für die Expresssion potentiell toxischer Proteine und ermöglicht eine kostengünstige Produktion insbesondere unter industriellen Bedingungen, da nur geringe L-Rhamnose-Konzentrationen erforderlich sind.

"Prokaryontische Wirtzelle" oder "prokaryontischer Wirtsorganismus" meint im Rahmen dieser Erfindung gram-positive oder gram-negative Bakterien, insbesondere solche gram-positive oder gram-negative Bakterien, die natürlicherweise in der Lage sind L-Rhamnose als Kohlenstoffquelle zu metabolisieren. L-Rhamnose kann von den meisten prokaryotischen Organismen als Kohlenstoffquelle genutzt werden.

Bevorzugt meint prokaryontische Wirtzelle oder prokaryontischer Wirtsorganismus alle Gattungen und Arten der Enterobacteriaceae und der Familien der Actinomycetales, ganz besonders bevorzugt die Enterobacteriaceae Arten Escherichia, Serratia, Proteus, Enterobacter, Klebsiella, Salmonella, Shigella, Edwardsielle, Citrobacter, Morganella, Providencia und Yersinia.

Ferner bevorzugt sind die Arten Pseudomonas, Burkholderia, Nocardia, Acetobacter, Gluconobacter, Corynebacterium, Brevibacterium, Bacillus, Clostridium, Cyanobacter, Staphylococcus, Aerobacter, Alcaligenes, Rhodococcus und Penicillium.

Am meisten bevorzugt sind Escherichia Arten, insbesondere Escherichia coli.

"L-Rhamnose-induzierbaren Promotor" meint allgemein all solche Promotoren die in Gegenwart von L-Rhamnose eine höhere Expressionsaktivität aufweisen, als in Abwesenheit von L-Rhamnose. Die Expression ist in Gegenwart von L-Rhamnose mindestens doppelt so hoch, bevorzugt mindestens fünfmal so hoch, ganz besonders bevorzugt mindestens zehnmal so hoch, am meisten bevorzugt mindestens einhundertmal so hoch wie in Abwesenheit von L-Rhamnose. Bevorzugt werden im Rahmen der Ermittlung der Expressionshöhe solche Nukleinsäuresequenzen in funktioneller Verknüpfung mit dem zu prüfenden Promotor eingesetzt, die für leicht quantifizierbare Proteine kodieren. Ganz besonders bevorzugt sind dabei Reporterproteine (Schenborn E, Groskreutz D (1999) Mol Biotechnol 13(1): 29-44) wie "green fluorescence protein" (GFP) (Chui WL et al. (1996) Curr Biol 6:325-330; Leffel SM et al.(1997) Biotechniques 23(5):912-8), Chloramphenicoltransferase, Luziferase (Millar et al. (1992) Plant Mol Biol Rep 10:324-414), β-Glucuronidase oder β-Galactosidase.

Dabei kann die Konzentration von L-Rhamnose in dem Medium allgemein in einem Bereich von ungefähr 0,0001 g/l bis ungefähr 50 g/l, bevorzugt 0,001 g/l bis 5 g/l, besonders bevorzugt 0,01 g/l bis 0,5 g/l liegen.

Insbesondere bevorzugt ist der rhaP_{BAD}-Promotor aus dem L-Rhamnose-Operon rhaBAD in E. coli (Egan & Schleif (1994) J Mol Biol 243:821-829), sowie seine funktionellen Äquivalente aus anderen prokaryontischen Organismen, insbesondere Organismen der Enterobacteriaceae Familie.

Ganz besonders bevorzugt sind Promotoren, die mindestens ein RhaS-Bindeelement gemäß SEQ ID NO: 5 oder ein funktionelles Äquivalent desselben als auch ein funktionell äquivalentes Fragment der vorgenannten enthalten.

Insbesondere bevorzugt sind Promotoren die eine Sequenz gemäß SEQ ID NO: 2, 3 oder 4 enthalten sowie funktionelle Äquivalente derselben als auch funktionell äquivalente Fragmente der vorgenannten.

Funktionelle Äquivalente zu einem Promotor umfassend eine Sequenz gemäß SEQ ID NO: 2, 3, 4 oder 5 umfassen bevorzugt solche Promotoren, die
a) im wesentlichen die gleiche Promotoraktivität wie der Promotor umfassend eine Sequenz gemäß SEQ ID NO: 2, 3, 4 oder 5 aufweisen und
b) eine Homologie von mindestens 50 %, bevorzugt 70 %, vorzugsweise mindestens 80 %, besonders bevorzugt mindestens 90 %, ganz besonders bevorzugt mindestens 95 %, am meisten bevorzugt 99% zu der Sequenz des besagten Promotors aufweisen, wobei sich die Homologie über eine Länge von von mindestens 30 Basenpaaren, bevorzugt mindestens 50 Basenpaaren, besonders bevorzugt von mindestens 100 Basenpaaren erstreckt.

Funktionelle Äquivalente zu einem Promotor umfassend eine Sequenz gemäß SEQ ID NO: 2, 3, 4 oder 5 meint insbesondere natürliche oder künstliche Mutationen des besagten Promotors sowie homologe Sequenzen und funktionell äquivalente Sequenzen aus anderen Organismen, bevorzugt aus anderen prokaryontischen Organismen, insbesondere Organismen der Enterobacteriaceae Familie, die im wesentlichen die gleiche Promotoraktivität wie der besagte Promotor aufweisen.

"Im wesentlichen die gleiche Promotoraktivität" meint die Induzierbarkeit der Expressionsaktivität durch L-Rhamnose nach der oben gegebenen allgemeinen Definition für L-Rhamnose-induzierbaren Promotoren.

Wie oben beschrieben, bindet das RhaR-Protein in Anwesenheit von L-Rhamnose an den rhaP_{RS}-Promotor und initiiert seine eigene Expression als auch die RhaS-Expression. RhaS wiederum bindet mit L-Rhamnose als Effektor an den rhaP_{BAD}-Promotor und aktiviert dann den rhaP_{BAD}-Promotor und somit die Transkription der durch besagten Promotor regulierten Nukleinsäuresequenzen. Dieser vorgeschaltete Regulationsapperat - bestehend aus RhaR, RhaS und dem rhaP_{RS}-Promotor - können durch den prokaryotischen Wirtsorganismus natürlicherweise bereitgestellt, durch gentechnische Verfahren in dessen Genom insertiert oder aber mittels des im Rahmen der Erfindung eingesetzten DNA-Konstruktes zur Verfügung gestellt werden. Eine in diesem Zusammenhang geeignete Promotorkassette ist die durch SEQ ID NO: 1 beschriebene Sequenz.

Sollte die für die Induktion in die Zelle erforderliche L-Rhamnose Aufnahme nicht ausreichen, kann es vorteilhaft sein in Organismen, die bespielsweise natürlicherweise keinen L-Rhamnose-Transporter exprimieren, diesen transgen zur Expression zu bringen. Bisherige Erfahrungen zeigen jedoch das der aktive Rhamnosetransport keine limitierende Größe für die Effizienz des erfindungsgemößen Expressionssystems darstellen sollte.

"L-Rhamnose-Isomerase" meint allgemein all solche Proteine, die befähigt sind, L-Rhamnose in eine andere Hexose zu konvertieren. Bevorzugt meint L-Rhamnose-Isomerase, solche Proteine, die befähigt sind L-Rhamnose in L-Rhamnulose zu überführen (EC 5.3.1.14). Besonders bevorzugt ist das RhaA-Gen aus Organismen der Enterobacteriaceae Familie, insbesondere E.coli. Am meisten bevorzugt meint L-Rhamnose-Isomerase das Protein gemäß SEQ ID NO: 9, sowie homologe Sequenzen aus anderen Organismen, bevorzugt aus anderen prokaryontischen Organismen.

Funktionelle Äquivalente zu der L-Rhamnose-Isomerase gemäß SEQ ID NO: 9 umfasst bevorzugt solche Sequenzen die
a) im wesentlichen die gleiche Enzymaktivität wie die L-Rhamnose-Isomerase gemäß SEQ ID NO: 9 aufweisen und
b) die eine Homologie aufweisen von mindestens 50 %, bevorzugt 70 %, vorzugsweise mindestens 80 %, besonders bevorzugt mindestens 90 %, ganz besonders bevorzugt mindestens 95 %, am meisten bevorzugt 99% zu der Sequenz der L-Rhamnose-Isomerase gemäß SEQ ID NO: 9, wobei sich die Homologie über eine Länge von von mindestens 30 Aminosäuren, bevorzugt mindestens 50 Aminosäuren, besonders bevorzugt von mindestens 100 Aminosäuren, ganz besonders bevorzugt von mindestens 200 Aminosäuren, am meisten ebvorzugt über die gesamte Länge des Proteins erstreckt.

Neben der L-Rhamnose-Isomerase können noch weitere Defizienzen in Bezug auf Gene vorliegen, die eine Funktion der L-Rhamnose-Metaboliserung haben. Insbesondere seien hierbei zu nennen Defizienz der Rhamnulose-1-Phosphatase/Kinase (z.B. RhaB; beispielsweise beschrieben durch SEQ ID NO: 11), eine Defizienz der Rhamnulophosphat-Aldolase (z.B. RhaD; beispielsweise beschrieben durch SEQ ID NO: 13) oder eine Defizienz in mindestens einem die Expression vorgenannter Proteine kontrollierenden regulatorischen Element (wie z.B. Promotor, Regulator o.ä.).

Unter Umständenkann es ferner vorteilhaft sein eine Defizienz in einem aktiven Rhamnose-Transportsystem (z.B. RhaT; beispielsweise beschrieben durch SEQ ID NO: 19) zu erzeugen.

"Defizienz" meint in Bezug auf eine L-Rhamnose-Isomerase oder ein anderes Enzym der L-Rhamnose-Aufnahme/Metabolisierung die im wesentlichen vollständige, auf unterschiedliche zellbiologische Mechanismen beruhende Unterbindung oder Blockierung der Expression des entsprechenden Zielgens oder der von ihm abgeleiteten mRNA und/oder des dadurch kodierten Proteinproduktes oder die Veränderung der Proteinsequenz des Genproduktes in einer Weise, das dessen Funktion und/oder Aktivität im wesentlichen unterbunden oder so geändert ist, dass L-Rhamnose im wesentlichen nicht mehr umgesetzt werden kann.

Eine Unterbindung oder Blockierung im Sinne der Erfindung umfasst insbesondere die mengenmässige Verringerung einer vom Zielgen exprimierten mRNA und/oder des dadurch kodierten Proteinproduktes bis hin zu einem im wesentlichen vollständigen Fehlen derselben. Dabei wird die Expression einer bestimmten mRNA und/oder des dadurch kodierten Proteinproduktes in einer Zelle oder einem Organismus im Vergleich zu der selben Zelle oder Organismus, die dem Verfahren nicht unterworfen wurden, bevorzugt um mehr als 50 %, besonders bevorzugt um mehr als 80%, ganz besonders bevorzugt um mehr als 90 %, am meisten bevorzugt mehr als 95 % vermindert.

Ganz besonders bevorzugt meint Verminderung die vollständige Inaktivierung eines endogenen Gens ("knockout"-Mutation).

Eine Unterbindung oder Blockierung kann auf unterschiedlichen Mechanismen beruhen. Bevorzugt beruht die Unterbindung oder Blokkierung auf einer Mutation in dem entsprechenden Zielgen, wobei die Mutation in einer Substitution, Deletion und/oder Addition eines oder mehrerer Nukleotide bestehen kann. Besonders bevorzugt ist eine Unterbindung oder Blockierung mittels Transposon unterstützter Mutagenese oder mittels gezieltem Knock-out.

Die Verminderung kann durch dem Fachmann geläufigen Verfahren ermittelt werden. So kann die Verminderung der Proteinmenge beispielsweise durch immunologischen Nachweis des Proteins bestimmt werden. Weiterhin können biochemische Techniken wie Northern-Hybridisierung, "nuclease protection assay", Reverse Transkription (quantitative RT-PCR), ELISA ("enzyme linked immunosorbent assay"), Western-Blotting, Radioimmunoassay (RIA) oder andere Immunoassays sowie "fluorescence activated cell analysis" (FACS) eingesetzt werden. Je nach Art des verminderten Proteinproduktes kann auch dessen Aktivität oder der Einfluss auf den Phänotyp des Organismus oder der Zelle ermittelt werden.

"Proteinmenge" meinte die Menge eines bestimmten Polypeptides in einem Organismus, einem Gewebe, einer Zelle oder einem Zellkompartiment.

"Verminderung" der Proteinmenge meint die Verminderung der Menge eines bestimmten Polypeptides in einem Organismus, einem Gewebe, einer Zelle oder einem Zellkompartiment im Vergleich zu dem Wildtyp derselben Gattung und Art auf den dieses Verfahren nicht angewendet wurde, unter ansonst gleichen Rahmenbedingungen (wie beispielsweise Kulturbedingungen, Alter, Nährstoffzufuhr etc.). Die Verminderung beträgt dabei mindestens 50 %, bevorzugt mindestens 70%, besonders bevorzugt mindestens 90 %, ganz besonders bevorzugt mindestens 95%, am meisten bevorzugt mindestens 99 %. Verfahren zur Bestimmung der Proteinmenge sind dem Fachmann bekannt. Beispielhaft seien zu nennen: Das Mikro-Biuret Verfahren (Goa J (1953) Scand J Clin Lab Invest 5:218-222), die Folin-Ciocalteu-Methode (Lowry OH et al. (1951) J Biol Chem 193:265-275) oder die Messung der Adsorption von CBB G-250 (Bradford MM (1976) Analyt Biochem 72:248-254).

Die Verminderung der L-Rhamnose-Isomerase Aktivität kann insbesondere mittels enzymatischer Testsysteme bestimmt werden. Entsprechende Testsysteme sind dem Fachmann bekannt (Bhuiyan SH et al. (1997) J Ferment Bioeng 84(4):319-323).

"In prokaryontischen Wirtszellen episomal replizierbares DNA-Konstrukt" meint all solche DNA-Konstrukte, welche unterschieden sind von der chromosomalen DNA der besagten Wirtszelle und parallel zu dieser in der besagten Wirtszelle existieren und befähigt sind in besagter Wirtzelle unter Verwendung zelleigener oder anderer (beispielsweise über das DNA-Konstrukt selber kodierter) Replikationsmechanismen zu replizieren. Das DNA-Konstrukt kann eine einzel- oder doppelsträngige DNA-Struktur darstellen. Bevorzugt hat das DNA-Konstrukt zumindest zeitweise (d.h. zu einem Zeitpunkt seines Replikationszyklus) eine doppelsträngige DNA-Struktur.

Bevorzugt liegen die besagten episomal replizierbaren DNA-Konstrukte in einer Kopienzahl von mindestens 1, bevorzugt mindestens 5, besonders bevorzugt mindestens 10 in einer Wirtszelle vor.

"Selektion prokaryontischer Wirtszellen, welche besagtes DNA-Konstrukt in episomaler Form enthalten" meint die Auswahl von Wirtzellen, die besagtes DNA-Konstrukt in episomaler Form enthalten. Die Auswahl kann beispielsweise unter Verwendung eines der unten beschriebenen Selektionsmarker realisiert werden. Bevorzugt insertiert das DNA-Konstrukt nicht in die chromosomale DNA der Wirtzelle. Dies kann beispielsweise dadurch verhindert werden, dass das DNA-Konstrukt keine Sequenzen aufweist, die über einen längeren Bereich identisch zu chromosomalen Sequenzen der Wirtzelle sind.

Bevorzugt haben besagte episomal replizierbaren DNA-Konstrukte eine Größe/Länge von maximal 100.000 Basen bzw. Basenpaaren, besonders bevorzugt maximal 50.000 Basen bzw. Basenpaaren, ganz besonders bevorzugt 10.000 Basen bzw. Basenpaaren (die Angabe Basen bzw. basenpaaren richtet sich danach, ob das DNA-Konstrukt eine einzel- oder doppelsträngige DNA-Struktur darstellt).

Bevorzugt handelt es sich bei dem DNA-Konstrukt um einen Vektor. Vektoren können beispielhaft Plasmide, Cosmide, Phagen, Viren, Retroviren oder auch Agrobacterien sein. Bevorzugt ist der Vektor eine zirkuläres Plasmid, das die zu exprimierende Nukleinsäuresequenz in rekombinanter Form umfasst und zu autonomer Replikation in der prokaryotischen Wirtzelle befähigt ist. Vektor kann im Rahmen dieser Erfindung auch als rekombinanter Vektor oder rekombinanter Expressionsvektor bezeichnet werden. Verschiedene Sequenzen, die die Replikation von DNA in Prokaryonten erlauben sind dem Fachmann bekannt. Beispielhaft seien genannt ORI (origin of DNA replication), der pBR322 ori oder der P15A ori (Sambrook et al.: Molecular Cloning. A Laboratory Manual, 2nd ed. Cold Spring Harbor Laboratory Press, Cold Spring Harbor, NY, 1989).

Entsprechend geeignete Replikationsursprünge, die eine geringe Kopienzahl gewährleisten ("Low-Copy") können aus BAC (Bacterial artificial chromosoms), F-Plasmiden, Cosmiden wie z.B. pWE15 isoliert werden. Entsprechend geeignete Replikationsursprünge, die eine mittlere Kopienzahl gewährleisten ("Medium-Copy") können z.B. aus pBR322 (Lin-Chao S, Bremer H, Mol Gen Genet 1986 203(1): 143-149) und Derivate wie der pJOE-Serie, pKK223-3, pQE30, pQE40 oder Plsamiden mit einem R1 Ursprung wie pRSF1010 und Derivate wie z.B. pML122, p15A, pSC101 isoliert werden. Entsprechend geeignete Replikationsursprünge, die eine hohe Kopienzahl gewährleisten ("High-Copy") können z.B. aus Phagemiden wie pBluescript II SK/KS+/-, pGEM etc. isoliert werden. Die jeweils in einer Zelle vorliegende Kopienzahl wird zum Teil durch den sogenannten Replikationsursprung (auch Replikon genannt) bestimmt. Plasmide der pBR322 Serien enthalten den ColE1 Replikationsursprung aus pMB1. Dieser ist relativ streng kontrolliert und resultiert in einer Kopienzahl von ca. 25 pro Zelle. Plasmide der pUC umfassen eine mutierte ColE1 Version und können in 200 bis 700 Plasmidkopien pro Zelle vorliegen. Einige Plasmide umfassen den p15a Replikationsursprung, der in einer geringen Kopienzahl resultiert.

Als Vektoren seinen beispielhaft zu nennen:
a) in E.coli sind bevorzugt pQE70, pQE60 und pQE-9 (QIAGEN, Inc.); pBluescript Vektoren, Phagescript Vektoren, pNH8A, pNH16a, pNH18A, pNH46A (Stratagene Cloning Systems, Inc.); ptrc99a, pKK223-3, pKK233-3, pDR540, pRIT5 (Pharmacia Biotech, Inc.); pLG338, pACYC184, pBR322, pUC18, pUC19, pKC30, pRep4, pHS1, pHS2, pPLc236, pMBL24, pLG200, pUR290, pIN-III¹¹³-B1, λgt11 oder pBdCI,
b) in Streptomyces sind bevorzugt pIJ101, pIJ364, pIJ702 oder pIJ361,
c) in Bacillus sind bevorzugt pUB110, pC194 oder pBD214,
d) in Corynebacterium pSA77 oder pAJ667,
oder Derivate der vorstehend genannten Plasmide. Die genannten Plasmide stellen eine kleine Auswahl der möglichen Plasmide dar. Weitere Plasmide sind dem Fachmann wohl bekannt und können beispielsweise aus dem Buch Cloning Vektors (Eds. Pouwels P. H. et al. Elsevier, Amsterdam-New York-Oxford, 1985, ISBN 0 444 904018) entnommen werden.

"Transformation" oder "transformiert" meint die Einführung genetischen Materials wie beispielsweise eines Vektors (z.B. ein Plasmid) in eine prokaryotische Wirtzelle. Dazu stehen dem Fachmann verschiedene weiter unten im Detail beschriebene Verfahren zu Verfügung. Eine prokaryotische Wirtzelle, in die besagtes genetisches Material eingeführt wurde, als auch die aus dieser Zelle resultierenden "Nachkommen" und Kolonien, die besagtes genetisches Material umfassen, werden als "Transformanten" bezeichnet.

"Transduktion" oder "transduziert" meint die Einführung genetischen Materials in eine prokaryotische Wirtzelle ausgehend von dem genetischen Material eines Bakteriophagen. Eine prokaryotische Wirtzelle, in die besagtes genetisches Material eingeführt wurde, als auch die aus dieser Zelle resultierenden "Nachkommen" und Kolonien, die besagtes genetisches Material umfassen, werden als "Transduktanten" bezeichnet.

"Rekombinantes Protein" meint jedes Proteinprodukt, dass ausgehend von der zu exprimierenden Nukleinsäureseuqnz unter funktioneller Kontrolle des L-Rhamnose-induzierbaren Promotors exprimiert werden kann und schließt Peptide, Polypeptide, Proteine, Oligoproteine und/oder Fusionsproteine ein. Bevorzugt meint "rekombinantes Protein" ein Protein mikrobiellen, bakteriellen, tierischen oder pflanzlichen Ursprungs.

"Fusionsproteine" meint eine Fusion aus dem gewünschten Protein und Leitsequenzen die eine Expression in bestimmten Kompartimenten (z.B. Periplasma oder Cytoplasma) der Wirtzelle oder in das umgebende Medium ermöglichen. Beispielhaft sei die pelB Leitsequenz zu nennen (US 5,576,195; US 5,846,818).

"Expressionskassette" meint jeweils die Kombination eines Promotors mit mindestens einer unter dessen Kontrolle transkribierbaren Nukleinsäuresequenz.

"Heterolog" meint in Bezug auf das Verhältnis des L-Rhamnose-induzierbaren Promotors und der unter Kontrolle besagten Promotors zu exprimierenden Nukleinsäuresequenz, bzw. eine Expressionskassette oder einen Expressionsvektor alle solche durch gentechnische Methoden zustande gekommene Konstruktionen, in denen entweder
a) mindestens eine der zu exprimierenden Nukleinsäuresequenzen, oder
b) mindestens einer der L-Rhamnose-induzierbaren Promotoren, der die Expression besagter zu exprimierender Nukleinsäuresequenz steuert, oder
c) (a) und (b)
sich nicht in ihrer natürlichen, genetischen Umgebung (beispielsweise an ihrem natürlichen chromosomalen Locus) befinden oder durch gentechnische Methoden modifiziert wurden, wobei die Modifikation beispielhaft Substitutionen, Additionen, Deletionen, Inversion oder Insertionen eines oder mehrerer Nukleotidreste umfassen kann.

Im erfindungsgemäßen Verfahren werden die erfindungsgemäßen prokaryontischen Wirtszellen in einem Medium, dass das Wachstum dieser Organismen ermöglicht, angezüchtet. Dieses Medium kann ein synthetisches oder ein natürliches Medium sein. Je nach Organismus werden dem Fachmann bekannte Medien verwendet. Für das Wachstum der Mikroorganismen enthalten die verwendeten Medien eine Kohlenstoffquelle, eine Stickstoffquelle, anorganische Salze und gegebenenfalls geringe Mengen an Vitamine und Spurenelemente.

Vorteilhafte Kohlenstoffquellen sind beispielsweise Polyole wie Glycerin, Zucker wie Mono-, Di- oder Polysaccharide wie Glucose, Fructose, Mannose, Xylose, Galactose, Ribose, Sorbose, Ribulose, Laktose, Maltose, Saccharose, Raffinose, Stärke oder Cellulose, komplexe Zuckerquellen wie Melasse, Zuckerphosphate wie Fructose-1,6-bisphosphat, Zuckeralkohole wie Mannit, Alkohole wie Methanol oder Ethanol, Carbonsäuren wie Citronensäure, Milchsäure oder Essigsäure, Fette wie Sojaöl oder Rapsöl, Aminosäuren wie ein Aminosäurengemisch beispielsweise sog. Casamino acids (Difco) oder einzelne Aminosäuren wie Glyzin oder Asparaginsäure oder Aminozucker, die letztgenannten können auch gleichzeitig als Stickstoffquelle verwendet werden. Besonders bevorzugt sind Polyole, insbesondere Glycerin.

Bevorzugt sollte das eingesetzte Medium als Basismedium keine L-Rhamnose enthalten, um eine möglichst dichte Regulation der Expression zu gewährleisten. Die L-Rhamnose wird dann im Bedarfsfall, zum gewünschten Zeitpunkt oder Zelldichte in der jeweils gewünschten Konzentration zugefügt.

Vorteilhafte Stickstoffquellen sind organische oder anorganische Stickstoffverbindungen oder Materialien, die diese Verbindungen enthalten. Beispiele sind Ammoniumsalze wie NH₄Cl oder (NH₄)₂SO₄, Nitrate, Harnstoff, oder komplexe Stickstoffquellen wie Maisquellwasser, Bierhefeautolysat, Sojabohnenmehl, Weizengluten, Hefeextrakt, Fleischextrakt, Caseinhydrolysat, Hefe oder Kartoffelprotein, die häufig auch gleichzeitig als Stickstoffquelle dienen können.

Beispiele für anorganische Salze sind die Salze von Calcium, Magnesium, Natrium, Kobalt, Molybdän, Mangan, Kalium, Zink, Kupfer und Eisen. Als Anion dieser Salze sind besonders das Chlor-, Sulfat- und Phosphation zu nennen. Ein wichtiger Faktor zur Steigerung der Produktivität im erfindungsgemässen Verfahren ist die Kontrolle der Fe²⁺⁻ oder Fe³⁺-Ionenkonzentration im Produktionsmedium.

Gegebenenfalls werden dem Nährmedium weitere Wachstumsfaktoren zugesetzt, wie beispielsweise Vitamine oder Wachstumsförderer wie Biotin, 2-KLG, Thiamin, Folsäure, Nicotinsäure, Pantothenat oder Pyridoxin, Aminosäuren wie Alanin, Cystein, Prolin, Asparaginsäure, Glutamin, Serin, Phenylalanin, Ornithin oder Valin, Carbonsäuren wie Citronensäure, Ameisensäure, Pimelinsäure oder Milchsäure, oder Substanzen wie Dithiothreitol.

Das Mischungsverhältnis der genannten Nährstoffe hängt von der Art der Fermentation ab und wird im Einzelfall festgelegt. Die Mediumkomponenten können alle zu Beginn der Fermentation vorgelegt werden, nachdem sie falls erforderlich getrennt sterilisiert oder gemeinsam sterilisiert wurden, oder aber je nach Bedarf während der Fermentation kontinuierlich oder diskontinuierlich nachgegeben werden.

Die Züchtungsbedingungen werden so festgelegt, dass die Organismen so wachsen, dass die bestmöglichen Ausbeuten (zu ermitteln beispielsweise durch die Aktivitätsmenge des exprimierten rekombinaten Proteins) erreicht werden. Bevorzugte Züchtungstemperaturen liegen bei 15 °C bis 40 °C. Besonders vorteilhaft sind Temperaturen zwischen 25 °C und 37 °C. Vorzugsweise wird der pH-Wert in einem Bereich von 3 bis 9 festgehalten. Besonders vorteilhaft sind pH-Werte zwischen 5 und 8. Im allgemeinen ist eine Inkubationsdauer von wenigen Stunden bis zu einigen Tagen bevorzugt von 8 Stunden bis zu 21 Tagen, besonders bevorzugt von 4 Stunden bis 14 Tagen ausreichend. Innerhalb dieser Zeit reichert sich die maximale Menge an Produkt im Medium an.

Wie Medien vorteilhaft optimiert werden können, kann der Fachmann beispielsweise dem Lehrbuch Applied Microbiol Physiology, "A Practical Approach (Eds. PM Rhodes, PF Stanbury, IRL-Press, 1997, Seiten 53 - 73, ISBN 0 19 963577 3) entnehmen.

Das erfindungsgemässe Verfahren kann kontinuierlich oder diskontinuierlich in batch- oder fed-batch-weise durchgeführt werden.

"Mutation" oder "Mutationen" meint die Substitution, Addition, Deletion, Inversion oder Insertionen eines oder mehrerer Aminosäurereste bzw. Basen/Basenpaare.

"Homologie" zwischen zwei Nukleinsäuresequenzen meint die Identität der Nukleinsäuresequenz über die jeweils angegebene Sequenzlänge, die durch Vergleich mit Hilfe des Programmalgorithmus GAP (Wisconsin Package Version 10.0, University of Wisconsin, Genetics Computer Group (GCG), Madison, USA; Altschul et al. (1997) Nucleic Acids Res. 25:3389ff) unter Einstellung folgender Parameter berechnet wird:

| | |
|---|---|
| Gap Weight: 50 | Length Weight: 3 |
| Average Match: 10 | Average Mismatch:0 |

Beispielhaft wird unter einer Sequenz, die eine Homologie von mindestens 50 % auf Nukleinsäurebasis mit der Sequenz SEQ ID NO: 2 aufweist, eine Sequenz verstanden, die bei einem Vergleich mit der Sequenz SEQ ID NO: 2 nach obigem Programmalgorithmus mit obigem Parametersatz eine Homologie von mindestens 50 % aufweist.

"Homologie" zwischen zwei Polypeptiden meint die Identität der Aminosäuresequenz über die jeweils angegebene Sequenzlänge, die durch Vergleich mit Hilfe des Programmalgorithmus GAP (Wisconsin Package Version 10.0, University of Wisconsin, Genetics Computer Group (GCG), Madison, USA) unter Einstellung folgender Parameter berechnet wird:

| | |
|---|---|
| Gap Weight: 8 | Length Weight: 2 |
| Average Match: 2,912 | Average Mismatch:-2,003 |

Beispielhaft wird unter einer Sequenz, die eine Homologie von mindestens 50 % auf Proteinbasis mit der Sequenz SEQ ID NO: 9 aufweist, eine Sequenz verstanden, die bei einem Vergleich mit der Sequenz SEQ ID NO: 9 nach obigem Programmalgorithmus mit obigem Parametersatz eine Homologie von mindestens 50 % aufweist.

Für eine optimale Expression heterologer Gene in Organismen kann es vorteilhaft sein, die Nukleinsäuresequenzen entsprechend der im Organismus verwendeten spezifischen "codon usage" zu verändern. Die "codon usage" lässt sich anhand von Computerauswertungen anderer, bekannter Gene des betreffenden Organismus leicht ermitteln.

Das DNA-Konstrukt, welches den L-Rhamnose-induzierbaren Promotor und die unter dessen Kontrolle zu exprimierenden Nukleinsäuresequenz umfasst, gewährleistet aufgrund einer funktionellen Verknüpfung von besagtem Promotor und besagter Nukleinsäuresequenz die Transkription und/oder Translation besagter Nukleinsäuresequenz.

Unter einer funktionellen Verknüpfung versteht man allgemein eine Anordnung in der eine genetische Kontrollsequenz ihre Funktion in Bezug auf die zu exprimierende Nukleinsäuresequenz ausüben kann. Funktion kann dabei beispielsweise die Kontrolle der Expression d.h. Transkription und/oder Translation der Nukleinsäuresequenz bedeuten. Kontrolle umfasst dabei beispielsweise die Initiierung, Steigerung, Steuerung oder Suppression der Expression d.h. Transkription und ggf. Translation. Unter einer funktionellen Verknüpfung versteht man zum Beispiel die sequentielle Anordnung eines Promoter, der zu exprimierenden Nukleinsäuresequenz und ggf. weiterer regulativer Elemente wie zum Beispiel einem Terminator derart, dass jedes der regulativen Elemente seine Funktion bei der Expression der Nukleinsäuresequenz erfüllen kann. Dem Fachmann sind verschiedene Wege bekannt, um zu einem der erfindungsgemässen DNA-Konstrukte zu gelangen. Die Herstellung kann mittels gängiger Rekombinations- und Klonierungstechniken realisiert werden, wie sie beispielsweise in T Maniatis, EF Fritsch und J Sambrook, Molecular Cloning: A Laboratory Manual, Cold Spring Harbor Laboratory, Cold Spring Harbor, NY (1989) sowie in TJ Silhavy, ML Berman und LW Enquist, Experiments with Gene Fusions, Cold Spring Harbor Laboratory, Cold Spring Harbor, NY (1984) und in Ausubel, FM et al., Current Protocols in Molecular Biology, Greene Publishing Assoc. and Wiley Interscience (1987) beschrieben sind.

Besagtes DNA-Konstrukt kann weitere Funktionselemente enthalten. Der Begriff der Funktionselemente ist breit zu verstehen und meint all solche Sequenzen, die einen Einfluss auf das Zustandekommen, die Vermehrung oder Funktion der erfindungsgemässen DNA-Konstrukte oder Organismen haben. Funktionselemente gewährleisten, verstärken, regulieren oder modifizieren zum Beispiel die Transkription und gegebenenfalls Translation in entsprechenden Witsorganismen.

Funktionselemente sind beispielsweise beschrieben bei "Goeddel; Gene Expression Technology: Methods in Enzymology 185, Academic Press, San Diego, CA (1990)" oder "Gruber and Crosby, in: Methods in Plant Molecular Biology and Biotechnolgy, CRC Press,Boca Raton, Florida, eds.:Glick and Thompson, Chapter 7, 89-108" sowie den dort aufgewiesenen Zitaten. Je nach nachstehend näher beschriebenen Wirtsorganismus oder Ausgangsorganismus, der durch Einbringen der Expressionskassetten oder Vektoren in einen genetisch veränderten oder transgenen Organismus überführt wird, eignen sich verschiedene Kontrollsequenzen.

"Genetische Kontrollsequenzen" umfassen beispielsweise die 5'-untranslatierte Region oder die nichtkodierende 3'-Region von Genen. "Genetische Kontrollsequenzen" meint ferner Sequenzen, die für Fusionsproteine bestehend aus einer Signalpeptidsequenz kodieren. Beispielhaft aber nicht einschränkend seien zu nennen:
a) Selektionsmarker
   Selektionsmarker sind in der Regel erforderlich, um erfolgreich transformierte Zellen zu selektionieren und den Verlust des DNA-Konstruktes aus der Wirtszelle im Laufe der Zeit und der Zellteilungen zu verhindern. Ein solcher Verlust kann insbesondere dann auftreten, wenn das durch die zu exprimierende Nukleinsäuresequenz kodierte rekombinante Protein einen toxischen Effekt auf den prokaryontischen Organismus hat. Der mit dem Expressionskonstrukt eingebrachte selektionierbaren Marker verleiht den erfolgreich transformierten Zellen eine Resistenz gegen ein Biozid (zum Beispiel ein Antibiotikum wie zum Beispiel Ampicillin, Kanamycin oder Hygromycin) verleiht. Beispielhaft als Selektionsmarker seien genannt:
   - Amp (Ampicillin-Resistenz; b-Lactamase)
   - Cab (Carbenicillin-Resistenz)
   - Cam (Chloramphenicol-Resistenz)
   - Kan (Kanamycin-Resistenz)
   - Rif (Rifampicin-Resistenz)
   - Tet (Tetracyclin-Resistenz)
   - Zeo (Zeocin-Resistenz)
   - Spec (Spectinomycin)

   Der Selektionsdruck wird durch entsprechende Mengen des Antibiotikums aufrechterhalten. Beispielhaft seien zu nennen: Ampicillin 100 mg/1, Carbenicillin 100 mg/1, Chloramphenicol 35 mg/l, Kanamycin 30 mg/l, Rifampicin 200 mg/l, Tetracyclin 12,5 mg/l, Spectinomycin 50 mg/l.
   Selektionsmarker umfassen ferner solche Gene und Genprodukte, die beispielsweise durch Komplementierung einer genetischen Defizienz in der Aminosäure- oder Nukleotidsynthese, eine Selektion einer entsprechend transformierten Wirtszelle ermöglichen. Dazu werden i.a. Medien eingesetzt, die besagte Aminosäure oder den besagten Nukleotidbaustein nicht enthalten. Verschiedene derartige Systeme sind dem Fachmann bekannt. Beispielhaft seien die Defizienzen in der Tryptophan (z.B. trpC), Leucin (z.B. leuB), Histidin (z.B: hisB) Biosynthese zu nennen, wie sie z.B. im E.coli Stamm KC8 (Clontech) vorliegen. Diese Defizienzen können u.a. durch die selektionierbaren Marker TRP1, Leu2 und HIS3 komplementiert werden.
b) Transkriptionsterminatoren
   Der Transkriptionsterminator vermindert eine ungewollte Transkription und erhöht die Plasmid- und mRNA Stabilität.
c) Shine-Dalgarno Sequenzen
   Eine Shine-Dalgarno (SD) Sequenz ist erforderlich für die Initiation der Translation und ist komplementär zum 3'-Ende der 16S ribosomalen RNA. Die Effizienz der Initiation der Translation am Start-Kodon hängt von der tatsächtlichen Sequenz ab. Eine geeignete Konsensussequenz für E.coli ist beispielhaft: 5'-TAAGGAGG-3'. Sie ist ca. 4 bis 14 Nukleotide stromaufwärts des Startkodon lokalisiert, wobei das Optimum bei 8 Nukleotiden liegt. Um die Ausbildung von Sekundärstrukturen zu vermeiden (welche die Expression reduzieren können), sollte diese Region bevorzugt reich an A/T-Nukleotiden sein.
d) Startkodon
   Das Startkodon ist der Initiationpunkt der Translation. In E. coli ist ATG das meist genutzte Startkodon; GTG kann alternativ auch genutzt werden.
e) "Tags" und Fusionsproteins
   N- or C-terminale Fusionen der zu exprimierenden rekombinanten Proteine mit kürzeren Peptiden ("Tags") oder anderen Proteinen (Fusionpartnern) können vorteilhaft sein. Sie können beispielsweise eine verbesserte Expression, Löslichkeit, Detektierbarkeit und Aufreinigung ermöglichen. Bevorzugt werden derartige Fusuionen mit Protease-Spaltsequenzen (z.B. für Thrombin oder Faktor X) kombiniert, die eine Entfernung des "Tags" bzw. des Fusionspartners nach der Expression und Aufreinigung ermöglichen.
f) Multiple Klonierungsregionen (Multiple cloning site; MCS) erlauben und erleichtern die Insertion einer oder mehrerer Nukleinsäuresequenzen.
g) Stop-Kodon / Translationsterminatoren
   Von den drei möglichen Stopp-Kodons ist TAA bevorzugt, da es bei TAG und TGA unter Umständen zu einem "Durchlesen" ("Read-Through") ohne Abbruch der Translation kommen kann. Es können auch mehrere Stopp-Kodons infolge eingesetzt werden um eine verläßliche Termination zu gewährleisten.
h) Reportergene
   Reportergene kodieren für leicht quantifizierbare Proteine, die über Eigenfarbe oder Enzymaktivität eine Bewertung der Transformationseffizienz, der Expressionshöhe, des Expressionsortes oder -zeitpunktes gewährleisten. Reportergene können z.B. für nachfolgende Proteine kodieren: Hydrolasen, Fluoreszenzproteine, Biolumineszproteine, Glucosidasen oder Peroxidasen. Bevorzugt sind Luciferasen, β-Galactosidasen, β-Glucuronidase, "Green Fluorescence Protein", Acetyl-, Phospo- oder Adenyltransferasen (siehe auch Schenborn E, Groskreutz D (1999) Mol Biotechnol 13(1):29-44).

Im Falle von Selektionsmarkern oder Reporterproteinen ist die für besagte Proteine kodierende Nuzkleinsäuresequenz bevorzugt mit einem in dem entsprechenden prokaryontischen Wirtorganismus funktionellen Promotor und ggf. weiteren Kontrollsequenzen funktionell zu einer Expresionskassette verknüpft. Vorteilhafte Promotoren und Kontrollsequenzen sind dem Fachmann allgemein bekannt. Beispielhaft seien Promotoren wie cos-, tac-, trp-, tet-, lpp-, lac-, lacIq-, T7-, T5-, T3-, gal-, trc-, ara-, SP6-, λ-PR- oder λ-PL-Promotor zu nennen.

Die Herstellung einer transformierten Wirtzelle oder eines transformierten Wirtsorganismus erfordert, dass die entsprechende DNA (beispielsweise eine der erfindungsgemäßen Expressionskassetten oder Vektoren) in die entsprechende Wirtszelle eingebracht wird. Für diesen Vorgang, der als Transformation bezeichnet wird, steht eine Vielzahl von Methoden zur Verfügung (siehe auch Keown et al. (1990) Methods in Enzymology 185:527-537). So kann die DNA beispielhaft direkt durch Mikroinjektion, Elektroporation oder durch Bombardierung mit DNA-beschichteten Mikropartikeln (biolistische Verfahren mit der Genkanone "particle bombardment") eingeführt werden. Auch kann die Zelle chemisch, zum Beispiel mit Polyethylenglycol, permeabilisiert werden, so dass die DNA durch Diffusion in die Zelle gelangen kann. Die DNA kann auch durch Fusion mit anderen DNA-enthaltenden Einheiten wie Minicells, Zellen, Lysosomen oder Liposomen erfolgen. Elektroporation ist eine weitere geeignete Methode zur Einführung von DNA, bei der die Zellen reversibel durch einen elektrischen Impuls permeabilisiert werden. Als bevorzugte allgemeine Methoden seien zu nennen Calciumphosphat vermittelte Transformation, DEAE-Dextran vermittelte Transformation, kationische Lipid-vermittelte Transformation, Elektroporation, Transduktion, Infektion. Derartige Verfahren sind dem Fachmann geläufig und beispielsweise beschrieben (Davis et al.(1986) Basic Methods In Molecular Biology; Sambrook J et al. (1989) Molecular cloning: A laboratory manual, Cold Spring Harbor Laboratory Press; Ausubel FM et al. (1994) Current protocols in molecular biology, John Wiley and Sons; Glover DM et al. (1995) DNA Cloning Vol.1, IRL Press ISBN 019-963476-9).

Transformierte Zellen d.h. solche, die die eingeführte DNA enthalten, können von untransformierten selektioniert werden, wenn ein selektionierbarer Marker Bestandteil der eingeführten DNA ist. Verschiedene Selektionsmarker sind oben beschrieben.

Das erfindungsgemäße Verfahren ist hinsichtlich der Art und Sequenz der zu exprimierenden Nuleinsäuresequenz bzw. des davon ausgehend exprimierten rekombinanten Proteins nicht eingeschränkt. Die unter Kontrolle der L-Rhamnose-induzierbaren Promotors zu exprimierenden Nukleinsäuresequenzen können vielfältiger Art sein. Expression meint in diesem Zusammenhang Transkription und gegebenenfalls Translation. Neben der Expression von Nukleinsäuresequenzen, welche für rekombinante Proteine kodieren, können auch Nukleinsäuresequenzen exprimiert werden, die beispielsweise die Transkription einer antisense-RNA bedingen und so die Expression eines endogenen Gens der prokaryontischen Wirtszelle vermindern. Sowohl Sequenzen prokaryontischen als auch eukaryontischen Ursprungs können exprimiert werden. Bevorzugt werden Sequenzen exprimiert die für rekombinante Proteine kodieren, die in einem größerem Umfang herzustellen sind. Beispielhaft jedoch nicht einschränkend seien zu nennen:
a) Enzyme, wie z.B. Chymosin, Proteasen, Polymerasen, Saccharidasen, Dehydrogenasen, Nukleasen, Glucanasen, Glucoseoxidase, α-Amylase, Oxidoreduktases (wie Peroxidasen oder Laccasen), Xylanasen, Phytasen, Cellulasen, Collagenasen, Hemicellulasen und Lipasen. Insbesondere bevorzugt sind
   - Enzyme wie sie in Waschmitteln oder anderen Detergentien genutzt werden wie beispielsweise Meerrettichperoxidase, Proteasen, Amylasen, Lipasen, Esterasen oder Cellulasen
   - Enzyme wie sie in der Lebensmittelindustrie genutzt werden wie Proteasen, Lipasen, Lactasen, b-Glucanase, Cellulasen oder Pectinasen
   - Enzyme wie sie in industriellen Verfahren eingesetzt werden wie Lipasen, α-Amylasen, Amiloglucosidasen, Glucoamylasen, Pullulanasen, Glucoseisomerasen,
   - Enzyme wie sie in industriellen Verfahren zur Herstellung von Chemikalien und Feinchemikalien eingesetzt werden wie Lipasen, Amidasen, Nitrilhydratasen, Esterasen oder Nitrilasen
   - Enzyme wie sie in der Tierernährung eingesetzt werden wie β-Glucanasen
   - Enzyme wie sie in der Papier- oder Lederindustrie eingesetzt werden wie Amylasen, Collagenasen, Cellulasen oder Xylanasen.
b) Säugerproteine wie besipielsweise Blutproteins (z.B.Serumalbumin, Faktor VII, Faktor VIII, Faktor IX, Faktor X, Gewebeplasminogenfaktor, Protein C, von Willebrand-Faktor, antiThrombin 111 oder Erythropoietin), "Colony Stimulating Factors" (CFS) (z.B. "Granulocyte colony-stimulating factor" (G-CSF), "Macrophage colony-stimulating factor" (M-CSF) oder "Granulocyte macrophage colony-stimulating factor" (GM-CSF)), Cytokine (z.B. Interleukine), Integrine, Addressine, Selectine, Antikörper oder Antikörperfragmente, Strukturproteine (z.B. Collagen, Fibroin, Elastin, Tubulin, Actin oder Myosin), Wachstumsfaktoren, Zellzyklusproteine, Impfstoffe, Fibrinogen, Thrombin, Insulinen.

Besonders bevorzugt kodiert die zu exprimierende Nukleinsäuresequenz für ein rekombinantes.Protein ausgewählt aus der Gruppe bestehend aus Chymosinen, Proteasen, Polymerasen, Saccharidasen, Dehydrogenasen, Nukleasen, Glucanasen, Glucoseoxidasen, α-Amylasen, Oxidoreduktasen, Peroxidasen, Laccasen, Xylanasen, Phytasen, Cellulasen, Collagenasen, Hemicellulasen, Lipasen, Lactasen, Pectinasen, Amyloglucosidasen, Glucoamylasen, Pullulanasen, Glucoseisomerasen, Nitrilasen, Esterasen, Nitrilhydratasen, Amidasen, Oxygenasen, Oxynitrilasen, Lyasen, Lactonasen, Carboxylasen, Collagenasen, Cellulasen, Serumalbuminen, Faktor VII, Faktor VIII, Faktor IX, Faktor X, Gewebeplasminogenfaktoren, Protein C, von Willebrand-Faktoren, antiThrombinen, Erythropoietinen, "Colony Stimulating Factors", Cytokinen, Interleukinen, Insulinen, Integrine, Addressine, Selectinen, Antikörpern, Antikörperfragmenten, Strukturproteinen, Collagen, Fibroinen, Elastinen, Tubulinen, Actinen, Myosinen, Wachstumsfaktoren, Zellzyklusproteinen, Impfstoffen, Fibrinogenen und Thrombinen.

In einer bevorzugen Ausführungform ist das rekombinante Protein eine Nitrilase, bevorzugt eine Nitrilase beschrieben durch eine Aminosäuresequenz, die kodiert wird durch eine Nukleinsäuresequenz ausgewählt aus der Gruppe
a) einer Nukleinsäuresequenz mit der in SEQ ID N0: 6 dargestellten Sequenz,
b) Nukleinsäuresequenzen, die sich als Ergebnis des degenerierten genetischen Codes von der in SEQ ID NO: 6 dargestellten Nukleinsäuresequenz ableiten,
c) Derivate der in SEQ ID NO: 6 dargestellten Nukleinsäuresequenz, die für Polypeptide mit der in SEQ ID NO: 7 dargestellten Aminosäuresequenzen kodieren und mindestens 35 % Homologie auf Aminosäureebene aufweisen, ohne dass die enzymatische Wirkung der Polypeptide wesentlich reduziert ist.

Ein weiterer Gegenstand der Erfindung betrifft die Verwendung der oben beschriebenen erfindungsgemässen Wirtszellen oder -organismen zur Herstellung von Nahrungs- oder Futtermitteln, Pharmazeutika oder Feinchemikalien. Feinchemikalien meint bevorzugt Proteine, Enzyme, Vitamine, Aminosäuren, Zucker, Fettsäuren, natürliche und synthetische Geschmacks-, Aroma- und Farbstoffe.

Ferner betrifft die Erfindung Verfahren zur Herstellung von rekombinanten Proteinen, Enzymen und anderen Feinchemikalien wie beispielweise Aldehyden, Ketonen oder Carbonsäuren (bevorzugt chiraler Carbonsäuren) unter Einsatz einer der erfindungsgemäßen prokaryontischen Wirtszellen oder einer Präparationen derselben. Die bevorzugten Proteine und Enzyme sind oben aufgeführt.

Dabei kann die prokaryontische Wirtzelle in einem wachsenden, ruhenden, immobilisierten oder aufgeschlossenen Zustand vorliegen. Unter aufgeschlossenen Zellen sind beispielsweise Zellen zu verstehen, die über eine Behandlung mit beispielsweise Lösungsmitteln durchlässig gemacht worden sind, oder Zellen die über eine Enzymbehandlung, über eine mechanische Behandlung (z.B. French Press oder Ultraschall) oder über eine sonstige Methode aufgebrochen wurden. Die so erhaltenen Rohextrakte sind für das erfindungsgemässe Verfahren vorteilhaft geeignet. Auch partiell gereinigte Enzympräparationen können für das Verfahren verwendet werden. Ebenfalls geeignet sind immobilisierte Mikroorganismen oder Enzyme, die vorteilhaft in der Reaktion Anwendung finden können.

Ein weiterer Gegenstand der Erfindung betrifft Verfahren zur Herstellung chiraler Carbonsäuren, wobei ein racemisches Nitril (oder alternativ dessen Vorstufen Aldehyd und Blausäure/Cyanidsalz) zu besagter chiraler Carbonsäure umgesetzt wird durch Behandlung mit einer prokarontische Wirtszelle, die zumindest defizient ist in Bezug auf eine L-Rhamnose-Isomerase und mindestens ein in besagter Wirtzelle replizierbares DNA-Konstrukt enthält, welches eine für eine Nitrilase kodierende Nukleinsäuresequenz unter transkriptioneller Kontrolle eines durch L-Rhamnose-induzierbaren Promotors umfaßt, wobei besagter Promotor in Bezug auf besagte Nukleinsäuresequenz heterolog ist.

Die für die Nitrilase kodierende Nukleinsäuresequenz ist bevorzugt aus der Gruppe der oben angegebenen für Nitrilasen kodierenden Sequenzen ausgewählt.

Chirale Carbonsäuren sind gesuchte Verbindungen für die organische Synthesechemie. Sie sind Ausgangsprodukte für eine Vielzahl von pharmazeutischen Wirkstoffen oder Wirkstoffen für den Pflanzenschutz. Chirale Carbonsäuren können zur klassischen Racematspaltung über Diastereomeresalze verwendet werden. So wird R-(-)-oder S-(-)-Mandelsäure beispielsweise zur Racematspaltung racemischer Amine eingesetzt. R-(-)-Mandelsäure wird ausserdem als Zwischenprodukt bei der Synthese genutzt.

In einer bevorzugten Ausführungsform werden die chiralen Carbonsäuren der allgemeinen Formel I ausgehend von einem racemischen Nitril der allgemeinen Formel II hergestellt. 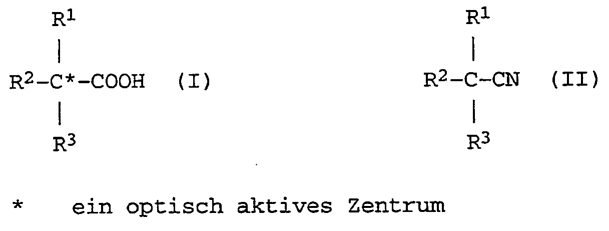
- R¹, R², R³: unabhängig voneinander Wasserstoff, substituiertes oder unsubstituiertes, verzweigtes oder unverzweigtes C1-C10-Alkyl-, C2-C10-Alkenyl-, substituiertes oder unsubstituiertes Aryl-, Hetaryl-, OR⁴ oder NR⁴R⁵ und wobei die Reste R¹, R² und R³ immer unterschiedlich sind,
- R⁴: Wasserstoff, substituiertes oder unsubstituiertes, verzweigtes oder unverzweigtes C1-C10-Alkyl-, C2-C10-Alkenyl-, C1-C10-Alkylcarbonyl-, C2-C10-Alkenylcarbonyl-, Aryl-, Arylcarbonyl-, Hetaryl- oder Hetarylcarbonyl-,
- R⁵: Wasserstoff, substituiertes oder unsubstituiertes, verzweigtes oder unverzweigtes C1-C10-Alkyl-, C2-C10-Alkenyl-, Aryl- oder Hetaryl-.

Als Nitril am meisten bevorzugt sind Mandelonitril, o-Chlormandelonitril, p-Chlormandelonitril oder m-Chlormandelonitril. Als chirale Carbonsäure sind am meisten bevorzugt R-Mandelsäure, S-Mandelsäure, R-p-Chlormandelsäure, S-p-Chlormandelsäure, R-m-Chlormandelsäure, S-m-Chlormandelsäure, R-o-Chlormandelsäure oder S-o-Chlormandelsäure.

Einzelheiten zu der Durchführung dieser Umsetzungen bzw. zur Aufreinigung der Produkte etc. sind beispielsweise in WO 00/23577 im Detail beschrieben. Auf die dort als beschriebenen Edukte, Produkte und Verfahrensparameter wird ausdrücklich bezug genommen.

### Beispiele

Allgemeine Nukleinsäureverfahren wie z.B. Klonierung, Restriktionsspaltungen, Agarose-Gelelektrophorese, Verknüpfen von DNA-Fragmenten, Transformation von Mikroorganismen, Anzucht von Bakterien und Sequenzanalyse rekombinanter DNA wurden wenn nichts anderes beschrieben wurde wie bei Sambrook et al. (1989) (Cold Spring Harbor Laboratory Press: ISBN 0-87969-309-6) beschrieben durchgeführt. Die Sequenzierung rekombinanter DNA-Moleküle erfolgte mit einem Laserfluoreszenz-DNA-Sequenzierer der Firma ABI nach der Methode von Sanger (Sanger et al. (1977) Proc Natl Acad Sci USA 74:5463-5467). Fragmente resultierend aus einer Polymerase Kettenreaktion wurden zur Vermeidung von Polymerasefehlern in zu exprimierenden Konstrukten sequenziert und überprüft.

### Beispiel 1: Charakterisierung des E.coli-Stammes JB1204

Escherichia coli JB1204 (CGSC6999, Bulawa CE & Raetz CRH (1984) J Biol Chem 259:11257-11264) besitzt laut Literatur eine Transposoninsertion "rha-14::Tn10", wobei zur Sequenz oder Funktion von "rha-14" keine genaueren Angaben gemacht wurden. JB1204 (ein K12-Derivat) ist aufgrund zahlreicher anderer Mutationen im Wachstum Stämmen wie TG1 und W3110 unterlegen, weshalb er selbst nicht zur technischen Proteinherstellung herangezogen wird.

Um zu testen, ob der Stamm E.coli JB1204 noch Rhamnose verstoffwechselt und ob die Induktion eines Rhamnose-abhängigen Expressionssystems in E. coli JB1204 beeinträchtigt ist, wurden kompetente JB1204-Zellen hergestellt und mit dem Plasmid pDHE1650 transformiert, das ein pJOE-Derivat ist und unter Kontrolle des Rhamnose-Promotors das Gen für eine Nitrilase trägt (Plasmid entspricht pDHE19.2 in DE 19848129). Nach 15 h Kultivierung bei 37 °C in LB-Ampicillin-Tetracyclin mit bzw. ohne Rhamnose wurde die optische Dichte der Kulturen gemessen und die Nitrilase-Aktivität nach Waschen der Zellen im "Resting-Cell"-Assay überprüft (siehe Tabelle 1). Bei Anzucht im Gegenwart von L-Rhamnose findet bei JB1204 wie auch beim Vergleichstamm TG1 eine Nitrilaseexpression statt, die ohne L-Rhamnose nicht erfolgt.

**Tab. 1**

| Probe | Rhamnosezusatz [g/L] | Rhamnoseverbrauch | OD₆₀₀ | Umsatz Mandelonitril |
|---|---|---|---|---|
| 1 | 2 | - | 5,9 | + |
| 1 | 0 | - | 5,7 | - |
| 2 | 2 | + | 11,9 | + |
| 2 | 0 | - | 8,0 | - |

| | | | | |
|---|---|---|---|---|
| 1, E.coli JB1204 pDHE1650 in LB Amp Tet; 2, E.coli TG1 pDHE1650 in LB Amp (Positivkontrolle) | | | | |

- Testbedingungen:: 10 mM Tris-HCl, 6 mM Mandelonitril, 40°C
- Analyse:: Probe mit 40 µl 1M HCl/ml abstoppen und nach Zellabtrennung mittels HPLC untersuchen wie in DE 19848129 beschrieben.

### Beispiel 2: Herstellung des Rhamnose-defizienten Wirtsstammes TG10 zur Produktion rekombinanter Proteine

Der für die Herstellung rekombinanter Biokatalysatoren genutzte Stamm TG1 wurde durch P1-Transduktion so modifiziert, dass er Rhamnose nicht mehr verstoffwechselt, aber das auf Rhamnose-Induktion basierende Expressionssystem der pJOE- und pDHE-Vektoren noch unbeeinträchtigt funktioniert (Bezeichnung dieses neuen Stammabkömmlings: TG10).

Um Fermentationsverfahren kostengünstig und mit hohen Ausbeuten fahren zu können, ist die Auswahl des E.coli-Stammes wichtig. Daher wurde als Wirtsstamm E. coli TG1 gewählt, der für produktive Hochdichtezellfermentationen bekannt ist (Korz et al. (1995) J Biotechnol 39:59-65). Die Rhamnose-Defizienz aus JB1204 wurde durch P1-Transduktion und Selektion auf Tetracyclin (15 µg/ml) auf TG1 pDHE1650 übertragen (=TG10 pDHE1650= Lu10569).

### 2.1 P1-Transduktionsprotokoll zum Transfer der Rhamnose-Defizienz von JB1204 (rha14::Tn10) auf TG1

### a) Herstellung des Donor-Lysates

- Den Donor, also JB1204 in 3 ml LB-Tet (15 µg/ml) ca. 15 h bei 37°C anziehen (Vorkultur).
- 3 ml LB-Tet + 5 mM CaCl₂ + 60 µl Vorkultur (=1:50) bis OD600= 0,3 - 0,5 bei 37°C inkubieren (ca. 45 min)
- + 100 µl (frisches) Lysat des Phagen P1, 10-120 min bis zur Zellyse gut weiterschütteln (Klärung, bei altem Lysat bis zu 5 h)
- + 60 µl Chloroform, 30 sec vortexen zur Abtötung restlicher Zellen, Lagerung bei 4°C.

### b) Infektion des Rezipienten

- Den Rezipienten, also TG1 pDHE1650 (=Lu9682) in 3 ml LB-Amp ca. 15 h bei 37°C anziehen (Vorkultur)
- 5 ml LB-Amp+ 5 mM CaCl₂ + 10 mM MgCl₂ + 10 mM MgSO₄ + 100 µl Vorkultur (=1:50) bis OD600= 0,3 - 0,5 bei 37 °C inkubieren (ca. 30 min), Rest Vorkultur auf Eis
- Vorkultur und Hauprkultur ernten, resuspendieren in 2,5 ml LB-Amp-Ca-Mg
- Je 2x 100 µl Rezipient mit mit 0, 5, 30, 100 µl Donor-Lysat versetzen sowie eine Kontrolle ohne Rezipient + 100 µl Donor-Lysat und 8 min bzw. 24 min *ohne zu schütteln* bei 30°C inkubieren (Infektion)
- + 100 µl 1 M Na-Citrat pH 7,0, 2 min bei 7000 rpm zentrifugieren, in 1 ml 0,1 M Citratpuffer pH 7,0 2-3x waschen und resuspendieren, 1 h 37°C *ohne zu schütteln*
- Ernte, resuspendieren in 100 µl 0,02 M Na-Citrat pH 7,0
- Je 80 µl ausplattieren auf LB-Amp-Tet sowie je 10 µl der Ansätze ohne Donorlysat-Zugabe auf LB-Amp, Inkubation Über Nacht bei 37°C
- Auf LB-Amp wird Rasen erhalten (Kontrolle). Kolonien von LB-Amp-Tet picken und Resistenzen, Rhamnose-Defizienz und -Induktionsfähigkeit, Aktivität verifizieren.

Parallel wurde auch TG1 pDHE1650 pAgro4 pHSG575, das Pendant zu TG1 pDHE1650 mit Chaperon-Coexpression (GroESL), transduziert (+Spectinomycin 50 µg/ml und Chloramphenicol 10 µg/ml im Medium; Bezeichnung TG10 pDHE1650 pAgro4 pHSG575=Lu10571).

Nach Übernachtkultivierung der erhaltenen Klone in 3 ml LB-Ampicillin-Rhamnose (ca. 2 g/l)-Medium (± Tetracyclin 10 µg/ml) wurden die optischen Dichten (λ=600 nm) der Kulturen bestimmt. Die HPLC-Analytik der Kulturüberstände zeigte, daß der erhaltene E. coli-Stamm TG10 pDHE1650 Rhamnose nicht verstoffwechseln kann. Die Zellen wurden anschließend in Puffer gewaschen und im Resting-Cell-Assay auf ihre Nitrilaseaktivität getestet (Tabelle 2).

Die Rhamnose-defizienten Klone zeigten eine ähnliche Nitril-verseifende Aktivität wie der entsprechender Vergleichsstamm (TG1pDHE1650). Die Rhamnose-Konzentration nahm bei den Klonen kaum ab.

**Tab. 2**

| Probe | Rhamnose Rest [g/L] | Zellkonz. [xfach] | Inkub.-zeit [min] | Säure [mM] | Aktivität (1x) [U/L] | OD₆₀₀ | Aktivität / OD₆₀₀ MW [U/L] |
|---|---|---|---|---|---|---|---|
| Blank | - | 0 | 60 | 0,01 | 0 | | |
| TG10 pDHE1650 | 1,71 | 0,01 | 60 | 1,02 | 1700 | 6,01 | 324 |
| | | 0,05 | 10 | 1,10 | 2200 | | |
| TG1 pDHE1650 | 0 | 0,01 | 60 | 0,84 | 1400 | 7,90 | 180 |
| | | 0,05 | 10 | 0,72 | 1440 | | |
| TG10 pDHE1650 pAgropHSG | 1,67 | 0,01 | 60 | 0,78 | 1300 | 5,01 | 295 |
| | | 0,05 | 10 | 0,83 | 1660 | | |
| TG1 pDHE1650 pAgropHSG | 0,34 | 0,01 | 60 | 1,18 | 1967 | 7,51 | 297 |
| | | 0,05 | 10 | 1,25 | 2500 | | |

- Testbedingungen:: 10 mM Tris-HCl, 6 mM Mandelonitril, 40°C
- Analyse:: Probe mit 40 µl 1M HCl/ml abstoppen und nach Zellabtrennung mittels HPLC untersuchen wie in DE 19848129 beschrieben (1U = 1 µmol Mandelsäure/min

### Beispiel 3: "Curing" des Rhamnose-defizienten Wirtsstamm TG10 pDHE1650

Die Durchführung der Transduktion mit E.coli TG1 pDHE1650 bot den Vorteil der Selektion gegen den Ursprungsstamm JB1204 mit Ampicillin. Für weitere Arbeiten wurde jedoch ein plasmidfreier Wirtsstamm benötigt, d.h. das Plasmid pDHE1650 sollte aus TG10 pDHE1650 entfernt weden ("Curing" von TG10 pDHE1650). Dazu wurde E.coli TG10 pDHE1650 von Eis in 3 ml LB-Tet ohne Ampicillin angeimpft und ÜN bei 37 °C inkubiert. Daraus wurden eine 3 ml-Hauptkulturen 1:100 in LB-Tet animpft, die einem Hitzeschock unterzogen wurden (2,5 min 42°C). Nach 16 h Schütteln bei 37°C betrug die OD₆₀₀ der Kultur 1,3 (entspricht ca. 1,3x10⁹ Zellen/ml). Je 100 µl der Verdünnungsstufen 10⁻⁴ bis 10⁻⁷ wurden ausplattiert auf LB-Tet und die erhaltenen Kolonien (560+140+15+0) überstempelt auf LB-Tet mit Ampicillin. Ein dort schwach gewachsener Klon wurde nochmals auf LB-Amp-Tet ausgestrichen. Er wuchs nicht auf LB-Amp-Tet und zeigte nach Minipräparation (LB-Tet-Anzucht) auch keine Plamid-DNA. Dieser Ampicillin-sensitive Klon wird als TG10 bezeichnet (=Lu10568) und dient als Ausgangsstamm für neue Überexpressionsstämme.

### Beispiel 4: Herstellung von rekombinanter L-Pantolacton-Hydrolase mit dem Rhamnose-defizienten Wirtsstamm E.coli TG10

Es wurden kompetente E. coli-TG10 Zellen hergestellt und mit dem Plasmid pDHE681, pAgro4 und pHSG575-transformiert (= Probe 1 in Tab.3). Nach Übernachtkultivierung bei 37 °C zeigten die Zellen eine hohe L-Pantolacton-verseifende Aktivität im Vergleich zum entsprechenden Kontrollstamm (TG1 pDHE681 pAgro4 pHSG575== Probe 2 in Tab.3), dessen maximale Aktivität i.d.R. nach 6-7 h Inkubation erreicht wird (ca. 1500 U/L) und durch längere Inkubation stark abfällt. Die Rhamnose (0,5 g/L) wurde von TG10 pDHE681 pAgro4 pHSG575 nicht verstoffwechselt.

**Tab. 3**

| Probe | Rhamnose Rest [g/L] | OD₆₀₀ | Zellkonz. [xfach] | Inkub.-zeit [h] | Säure [mM] | Aktivität (1x)[U/L] | Aktivität / OD₆₀₀ [U/L] |
|---|---|---|---|---|---|---|---|
| Blank | - | | 0 | 1,0 | 1,74 | - | |
| 1 | 0,52 | 6,35 | 0,2 | 1,0 | 29,9 | 2344,2 | 369,2 |
| 2 | 0 | 6,64 | 0,2 | 1,0 | 6,27 | 377,5 | 56,9 |

| | | | | | | | |
|---|---|---|---|---|---|---|---|
| 1, TG10 pDHE681 pAgro4 pHSG575; LB mit Ampicillin (Amp; 100 µg/ml) Tetracyclin (Tet 10 µg/ml), L-Rhamnose (Rha 0,5 g/l) und Isopropylthiogalactosid (IPTG 0,15 mM) 2, TG1 pDHE681 pAgro4 pHSG575; LB mit Ampicillin (Amp; 100 µg/ml), L-Rhamnose (Rha 0,5 g/l) und Isopropylthiogalactosid (IPTG 0,15 mM) | | | | | | | |

Der Test wurde detailierter wiederholt. Die Zugabe von Tetracyclin (15 µg/ml) zum Medium ist zur Erhaltung der Rhamnose-Defizienz nicht notwendig.

### Beispiel 5: Bestimmung der Abhängigkeit der Induktion von der L-Rhamnose-Konzentration

Der Stamm E.coli TG10 (pDHE1650, pAgro4, pHSG575) wurde analog zu Beispiel 1 auf LB Ampicillin (100 mg/l), Chloramphenicol 10 mg/l, Spectionomycin (50 mg/l), IPTG 0,15mM in Gegenwart verschiedener Rhamnosemengen (0 bis 2 g/l Rhamnose) angezogen und auf seine spezifische Nitrilaseaktivität hin untersucht (Doppelbestimmung). Bereits eine Konzentration von 0,01 g/l L-Rhamnose ergibt eine im Durchschnitt signifikante Induktion der Expression, während in Abwesenheit von Rhamnose keinerlei signifikante Expression (über die Enzymaktivität) ermittelt werden konnte.

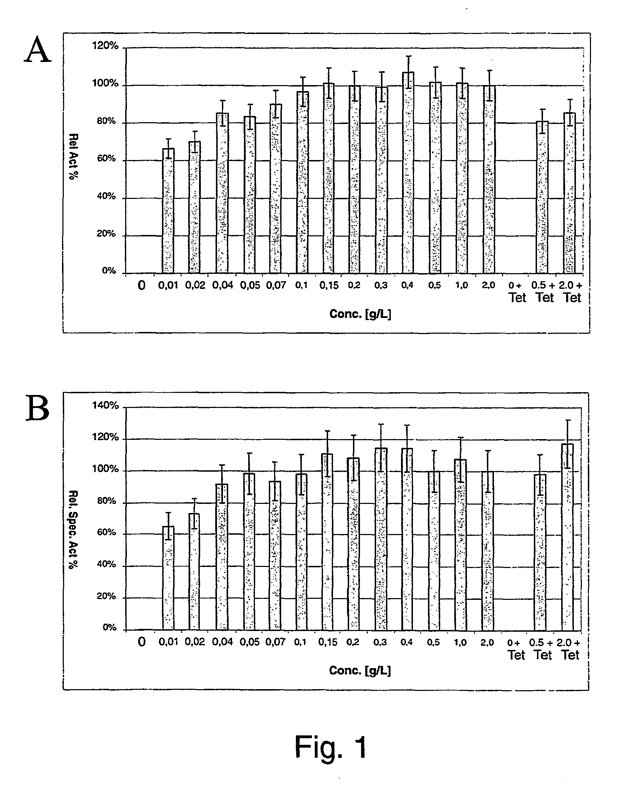

### Vgl. auch Fig.1:

- A:: Darstellung der relativen Aktivität (Rel. Act. %) im Verhältnis zu der L-Rhamnose-Konzentration (Conc. in g/l)
- B:: Darstellung der relativen spezifischen Aktivität (Rel. Spec Act. %) im Verhältnis zu der L-Rhamnose-Konzentration (Conc. in g/l)

**Tab. 4:**

| Rhamnosekonz. | OD600 | Rel. Aktiv. | Rel. spez. Akt. [g/l] |
|---|---|---|---|
| 0,00 | 5,4 | 0,1% | 0,1% |
| 0,01 | 6,2 | 66% | 65% |
| 0,02 | 5,8 | 70% | 73% |
| 0,04 | 5,7 | 85% | 92% |
| 0,05 | 5,2 | 83% | 98% |
| 0,07 | 5,9 | 90% | 93% |
| 0,10 | 6,0 | 97% | 98% |
| 0,15 | 5,6 | 101% | 111% |
| 0,20 | 5,6 | 100% | 108% |
| 0,30 | 5,3 | 99% | 115% |
| 0,40 | 5,7 | 107% | 114% |
| 0,50 | 6,2 | 102% | 100% |
| 1,00 | 5,8 | 101% | 108% |
| 2,00 | 6,1 | 100% | 100% |
| 0 + Tet | 4,7 | 0% | 0% |
| 0,5 + Tet | 5,1 | 81% | 98% |
| 2,0 + Tet | 4,5 | 86% | 117% |

### Beispiel 6: Analyse des Integrationsortes des Transposon im L-Rhamnose-Isomerase defizienten Stamm E.coli TG10

Um den Integrationsort des Transposons Tn10 näher zu charakterisieren, wurden die Rhamnose-Gene rhaT, rhaB, rhaA und rhaD via PCR (Pfu-Polymerase) im Vergleich von TG1 (pDHE681) und TG10 (pDHE681) untersucht. Bei Amplifikation von rhaA (L-Rhamnose-Isomerase) oder der Region rhaA-rhaD mit den Primern MKe259/260 bzw. MKe 258/259 wurde bei dem mutagenisierten Stamm TG10 im Unterschied zum Wildtypstamm TG1 keine spezifisches Amplifikat erhalten erhalten.
MKe258 5'-CCCAAGCTTGGATCATGTTTGCTCCTTACAG (rhaD 3'Ende + HindIII)
MKe259 5'-GCGAATTCGCATGACCACTCAACTGGAACA (rhaA 5'Ende + EcoRI)
MKe260 5'-CCCAAGCTTACCCGCGGCGACTCAAAATTT (rhaA 3'Ende + HindIII)

### Beispiel 7: Herstellung eines L-Rhamnose-Isomerase defizienten E.coli Stamms mittels gezieltem Knockout

Zur Inaktivierung der L-Rhamnose-Isomerase (rhaA) wird das rhaA-Gen zunächst mit den Primern MKe001 und MKe002 amplifiziert und in pBluescriptSK⁺ kloniert (XbaI/HindIII-Verdau und Ligation). Anschließend wird durch Restriktionsverdau mit BamHI und Auffüllreaktion mit Klenow-Fragment sowie anschließender Ligation ein Frameshift eingeführt und das entsprechende rha*-Fragment in den Gene-Replacement-Vektor pK03 (Link et al. (1997) J Bacteriol 179:6228-6237) umkloniert. Der Knock out des rhaA-Gens in TG1pDHE1650 durch homologe Rekombination mit dem rha*-Konstrukt wird nach Link et al. ((Link et al. (1997) J Bacteriol 179:6228-6237)) mittels Selektion auf Chloramphenicol bei 43°C, Replikaplattierung auf Saccharose bei 30°C und anschließender Verifizierung auf McConkey-Agar mit 1 g/L Rhamnose durchgeführt.
MKe001: 5'-ATAAGAATGCGGCCGCATGACCACTCAACTGGAACA-3'
MKe002: 5'-CTAGCTCTAGATTACCCGCGGCGACTCAA-3'

### Beispiel 8: Herstellung von rekombinanter Nitrilase mit dem Rhamnose-defizienten Wirtsstamm TG10

Die Fed-batch-Fermentation von TG10-Derivaten wie TG10 pDHE1650 pAgro4 pHSG575 erfolgt auf einem modifizierten Riesenberg-Medium mit Glycerin als C-Quelle und Rhamnose als Induktor zur Überexpression des Zielproteins, hier der Nitrilase. Mit dem Stamm wurden vergleichbare und höhere Zelldichten und Enzymaktivitäten erreicht.

### 8.1 Fermentation von E. coli TG 1

Die Fermentation des *Escherichia coli* (TG1 pDHE1650 pAgro4 pHSG575) erfolgte im 20 L Bioreaktor. Der Reaktor mit 10L Arbeitsvolumen wurde mit 200 ml Vorkultur aus Schüttelkolben angeimpft. Das Vorkulturmedium entspricht dem Hauptkulturmedium.

### Medium:

| | |
|---|---|
| 40 g | Glycerin 99,5 % |
| 15 g | Trypton |
| 13,3 g | Kaliumdihydrogenphosphat |
| 5 g | Hefeextrakt |
| 4 g | Di-Ammoniumhydrogenphosphat |
| 1,7 g | Citronensäure |
| 1,1 g | Magnesiumsulfat Heptahydrat |
| 1 mL | Spurenelementlösung SL Korz 1000 C |
| 0,1 mL | Tego KS 911 Antischaummittel |
| 0.062 g | Eisen(II)sulfat Heptahydrat |
| 10 mg | Thiaminhydrochlorid |
| ad 1 L. | VE-Wasser |

Das Medium wird 30 min bei 121°C sterilisiert. Anschließend werden 0,1 g Ampicilin steril zugesetzt

### Spurenelementlösung

| | |
|---|---|
| Citronensäure*H20 | 20 g |
| Kobalt(II)chlorid Hexachlorid (CoCl₂ * 6H₂O) | 2,5 g |
| Mangan(II)chlorid Tetrachlorid (MnCl₂ * 4H₂O) | 3,0 g |
| Kupfer(II)chlorid Dihydrat (CuCl₂ * 2H₂O) | 0,3 g |
| Borsäure (H₃BO₃) | 0,6 g |
| Natriummolybdat Dihydrat (Na₂MoO₄ * 2H₂O) | 0,5 g |
| Zinkacetat Dihydrat (Zn(CH3COO)₂ * 2H₂O) | 2,6 g |
| ad 1L VE- H2O | |

### Glycerinfeedlösung

| | |
|---|---|
| 2 L | VE-Wasser |
| 211 g | Natriumsulfat |
| 13,6 g | Eisen(II)sulfat Heptahydrat |
| 8,8 kg | Glycerin 99,5 % |
| 220 mL | Spurenelementlösung |

### Rhamnosefeedlösung

| | |
|---|---|
| 703 g | VE-Wasser |
| 297 g | Rhamnose Monohydrat |

Die Fermentation erfolgt bei einer Temperatur von 37°C. Die Begasung wird zwischen 8-30 L/min, die Rührerdrehzahl von 400 bis 1500 1/min geregelt um einen pO₂ von 20 % nicht zu unterschreiten. Nach 1 h Fermentationszeit wird die Kultur mit IPTG (0,15 mM) induziert. Anschließend werden 76 ml Rhamnosefeedlösung zugesetzt. Bei einem Unterschreiten der Rhamnosekonzentration im Fermenter von 1.0 g/L wird Rhamnosefeedlösung nachdosiert. Nach Verbrauch der vorgelegten Glycerinmenge wird kontinuierlich Glycerin zugefüttert.

### Ergebnisse:

| Zeit | p02 | BTM | Rhamnose | dosierte Rhamnosefeedlösung | Glycerin |
|---|---|---|---|---|---|
| [h] | [%] | [g/L] | [g/L] | [g] | [g/L] |
| 0 | 0 | 0 | 0 | 0 | 40.0 |
| 2 | 75.8 | 2.3 | 1.70 | 76 | 35.9 |
| 5 | 20.5 | 7.5 | 1.54 | 115 | 33.6 |
| 8 | 33.7 | 17.3 | 1.96 | 244 | 25.4 |
| 11 | 39.3 | 15.7 | 3.11 | 365 | 17.0 |
| 14 | 22.6 | 18.8 | 2.71 | 364 | 8.6 |
| 17 | 30.1 | 21.4 | 1.87 | 404 | 0 |
| 20 | 35.1 | 24.8 | 1.36 | 474 | 0 |
| 23 | 21.5 | 31.8 | 1.18 | 673 | 0 |
| 26 | 23.9 | 28.7 | 1.80 | 970 | 0 |
| 29 | 36.4 | 42.2 | 0.48 | 1234 | 0 |
| 32 | 28.5 | 38.7 | 1.20 | 1639 | 0 |
| 35 | 29.8 | 47.0 | 1.22 | 2033 | 0 |
| 38 | 44.3 | 49.2 | 1.19 | 2474 | 0 |
| 41 | 47.6 | 45.4 | 1.45 | 2879 | 0 |
| 44 | 46.2 | 45.2 | 1.80 | 3237 | 0 |
| Aktivität nach 44h: 57200 U/L | | | | | |

### 8.2 Fermentation von E. coli TG 10

Die Fermentation des *Escherichia coli* TG10 (pDHE1650 pAgro4 pHSG575) erfolgte nach derselben Vorschrift wie in Beispiel 1 mit dem Unterschied, dass die Induktion mit 18,5 g Rhamnosefeedlösung vorgenommen wurde. Es wurde keine Nachdosierung der Rhamnose vorgenommen.

### Ergebnisse:

| Zeit | p02 | BTM | Rhamnose | dosierte Rhamnosefeedlösung | Glycerin |
|---|---|---|---|---|---|
| [h] | [%] | [g/L] | [g/L] | [g] | [g/L] |
| 0 | 0 | 0 | 0.00 | 0 | 40.0 |
| 2 | 71.4 | 2.7 | 0.58 | 18.5 | 38.6 |
| 5 | 20.7 | 7.0 | 0.59 | 18.5 | 36.5 |
| 8 | 21.7 | 13.2 | 0.59 | 18.5 | 26.4 |
| 11 1 | 31.1 | 16.9 | 0.57 | 18.5 | 13.2 |
| 14 | 44.6 | 19.0 | 0.60 | 18.5 | 0 |
| 17 | 50.5 | 24.0 | 0.58 | 18.5 | 0 |
| 20 | 35.9 | 26.1 | 0.57 | 18.5 | 0 |
| 23 | 33.9 | 33.4 | 0.58 | 18.5 | 0 |
| 26 | 40.4 | 36.0 | 0.57 | 18.5 | 0 |
| 29 | 38.2 | 40.8 | 0.55 | 18.5 | 0 |
| 32 | 34.3 | 45.3 | 0.58 | 18.5 | 0 |
| 35 | 45.7 | 48.7 | 0.50 | 18.5 | 0 |
| 38 | 40.0 | 50.7 | 0.50 | 18.5 | 0 |
| 41 | 31.8 | 52.5 | 0.44 | 18.5 | 0 |
| 44 | 29.5 | 50.0 | 0.44 | 18.5 | 0 |
| Aktivität nach 44h: 59200 U/L | | | | | |

### 8.3 Aktivitätstest:

Zu 880 µl Natrium-Kalium-Phosphatpuffer (10mM) werden 50 µl Zellsuspension pipettiert und auf 30°C temperiert. Die Reaktion wird durch Zugabe von 20 µl methanolischer Mandelonitrillösung (12%) gestartet. Nach 10min wird er Enzymreaktion durch Zugabe von 50 µl 1M HCl gestoppt. Die Zellmasse wird abzentrifugiert und die Mandelsäurekonzentration im Überstand wird per HPLC (ODS Hypersil 100*2,0 mm, Laufmittel: 75% H3PO4 (14.8mM) / 25% Methanol; Flußrate: 0.5 ml/min; Injektionvolumen: 2 µl; Säulentemperatur: 40°C; Detektion: 210nm; Retentionzeit Mandelsäure: 0.9 min) gemessen.

### 8.4 Bestimmung der Rhamnosekonzentration:

Zur online Probenahme am Fermenter bedient man sich eines Keramikfilters und einer kontinuierlich betriebenen Schlauchpumpe. Die Programmierung der HPLC-Anlage erfolgt so, daß nach jeder abgeschlossenen Analyse eine erneute Einspritzung erfolgt. Dazwischen wird das Filtrat aus dem Fermenter in ein Abfallgefäß gepumpt.

### Chromatographische Bedingungen:

| | |
|---|---|
| Säule: | HPX 87 H, 7,8 x 300 mm |
| Eluent: | 0,005 M H₂SO₄ |
| Flußrate: | 0,5 mL/min |
| Injektionsvolumen: | 1 µL |
| Säulentemperatur: | 55°C |
| Detektion: | RI |

## Patentansprüche

1. Verfahren zur Expression von Nukleinsäuresequenzen in prokaryontischen Wirtszellen, wobei man
a) mindestens ein in besagten Wirtszellen episomal replizierbares, DNA-Konstrukt umfassend eine zu exprimierende Nukleinsäuresequenz unter transkriptioneller Kontrolle eines L-Rhamnose-induzierbaren Promotors, wobei besagter Promotor in Bezug auf besagte Nukleinsäuresequenz heterolog ist, in besagte Wirtzellen einbringt und
b) prokaryontischen Wirtszellen selektioniert, welche besagtes DNA-Konstrukt in episomaler Form enthalten und
c) die Expression besagter Nukleinsäuresequenz durch Zugabe von L-Rhamnose zu einer Kultur besagter selektionierter Wirtzellen induziert,
**dadurch gekennzeichnet, dass** die prokaryontische Wirtszelle zumindest defizient ist in Bezug auf L-Rhamnose-Isomerase.

2. Verfahren nach Anspruch 1, wobei die prokaryontische Wirtzelle ausgewählt ist aus den Arten der Familie der Enterobacteriaceae oder der Ordnung Actinomycetales.

3. Verfahren nach einem der Ansprüche 1 oder 2, wobei die prokaryontische Wirtzelle Escherichia coli ist.

4. Verfahren nach einem der Ansprüche 1 bis 3, wobei der L-Rhamnose-induzierbare Promotor der rhaP_{BAD}-Promotor aus E. coli oder ein funktionelles Äquivalent desselben oder ein funktionell äquivalentes Fragment der vorgenannten ist.

5. Verfahren nach einem der Ansprüche 1 bis 4, wobei der L-Rhamnose-induzierbare Promotor zumindest ein RhaS-Bindeelement gemäß SEQ ID NO: 5 oder ein funktionelles Äquivalent desselben oder ein funktionell äquivalentes Fragment der vorgenannten enthält.

6. Verfahren nach einem der Ansprüche 1 bis 5, wobei der L-Rhamnose-induzierbare Promotor mindestens eine Sequenz beschrieben durch SEQ ID NO: 1, 2, 3 oder 4 enthält.

7. Verfahren nach einem der Ansprüche 1 bis 6, wobei die L-Rhamnose-Isomerase durch die Aminosäuresequenz gemäß SEQ ID NO: 9 oder ein funktionelles Äquivalent derselben beschrieben ist.

8. Verfahren nach einem der Ansprüche 1 bis 7, wobei das episomal replizierbare DNA-Konstrukt eine Größe von maximal 100000 Basen bzw. Basenpaaren hat.

9. Verfahren nach einem der Ansprüche 1 bis 8, wobei das episomal replizierbare DNA-Konstrukt ausgewählt ist aus der Gruppe bestehend aus zirkulären Plasmidvektoren, Phagemiden und Cosmiden.

10. Verfahren nach einem der Ansprüche 1 bis 9, wobei die prokaryontische Wirtzelle mindestens eine weitere Defizienz in Bezug auf ein Gene aufweist, das eine Funktion der Rhamnose-Metaboliserung hat, wobei besagtes Gen für ein Protein kodiert ausgewählt aus der Gruppe bestehend aus der Rhamnulose-1-Phosphatase (RhaB) und der Rhamnulosephosphat-Aldolase (RhaD).

11. Verfahren nach einem der Ansprüche 1 bis 10, wobei die Expression der zu exprimierenden Nukleinsäuresequenz die Produktion eines durch besagte Nukleinsäuresequenz kodierten Proteins bedingt.

12. Verfahren nach einem der Ansprüche 1 bis 11, wobei die zu exprimierende Nukleinsäuresequenz für ein rekombinantes Protein kodiert ausgewählt aus der Gruppe bestehend aus Chymosinen, Proteasen, Polymerasen, Saccharidasen, Dehydrogenasen, Nukleasen, Glucanasen, Glucoseoxidasen, α-Amylasen, Oxidoreduktasen, Peroxidasen, Laccasen, Xylanasen, Phytasen, Cellulasen, Collagenasen, Hemicellulasen, Lipasen, Lactasen, Pectinasen, Amyloglucosidasen, Glucoamylasen, Pullulanasen, Glucoseisomerasen, Nitrilasen, Esterasen, Nitrilhydratasen, Amidasen, Oxygenasen, Oxynitrilasen, Lyasen, Lactonasen, Carboxylasen, Collagenasen, Cellulasen, Serumalbuminen, Faktor VII, Faktor VIII, Faktor IX, Faktor X, Gewebeplasminogenfaktoren, Protein C, von Willebrand-Faktoren, antiThrombinen, Erythropoietinen, "Colony Stimulating Factors", Cytokinen, Interleukinen, Insulinen, Integrine, Addressine, Selectinen, Antikörpern, Antikörperfragmenten, Strukturproteinen, Collagen, Fibroinen, Elastinen, Tubulinen, Actinen, Myosinen, Wachstumsfaktoren, Zellzyklusproteinen, Impfstoffen, Fibrinogenen und Thrombinen.

13. Prokaryontische Wirtszelle, die zumindest defizient ist in Bezug auf L-Rhamnose-Isomerase und mindestens ein in besagter Wirtzelle replizierbares DNA-Konstrukt enthält, welches eine zu exprimierende Nukleinsäuresequenz unter transkriptioneller Kontrolle eines durch L-Rhamnose-induzierbaren Promotors umfaßt, wobei besagter Promotor in Bezug auf besagte Nukleinsäuresequenz heterolog ist.

14. Verwendung einer prokarontischen Wirtszellen nach Anspruch 13 zur Herstellung von Nahrungs- oder Futtermitteln, Enzymen, Chemikalien, Pharmazeutika oder Feinchemikalien.

15. Verfahren zur Herstellung von rekombinanten Proteinen, Enzymen und Feinchemikalien unter Einsatz einer prokaryontischen Wirtszellen gemäß Anspruch 13 oder einer Präparationen derselben.

## Claims

1. A method for expressing nucleic acid sequences in prokaryotic host cells, where
a) at least one DNA construct which is capable of episomal replication in said host cells and which comprises a nucleic acid sequence to be expressed under the transcriptional control of an L-rhamnose-inducible promoter, where said promoter is heterologous with regard to said nucleic acid sequence, is introduced into said host cells and
b) prokaryotic host cells which comprise said DNA construct in episomal form are selected and
c) the expression of said nucleic acid sequence is induced by addition of L-rhamnose to a culture of said selected host cells,
wherein the prokaryotic host cell is at least deficient with regard to L-rhamnose isomerase.

2. The method according to claim 1, wherein the prokaryotic host cell is selected from the species of the family Enterobacteriaceae or the order Actinomycetales.

3. The method according to claim 1 or 2, wherein the prokaryotic host cell is Escherichia coli.

4. The method according to any of claims 1 to 3, wherein the L-rhamnose-inducible promoter is the rhaP_{BAD} promoter from E. coli or a functional equivalent thereof or a functionally equivalent fragment of the above promoters.

5. A method according to any of claims 1 to 4, wherein the L-rhamnose-inducible promoter comprises at least one RhaS binding element as shown in SEQ ID NO: 5 or a functional equivalent thereof or a functionally equivalent fragment of the above elements.

6. A method according to any of claims 1 to 5, wherein the L-rhamnose-inducible promoter comprises at least one sequence described by SEQ ID NO: 1, 2, 3 or 4.

7. The method according to any of claims 1 to 6, wherein the L-rhamnose isomerase is described by the amino acid sequence as shown in SEQ ID NO: 9 or a functional equivalent thereof.

8. The method according to one of claims 1 to 7, wherein the DNA construct which is capable of episomal replication has a size of not more than 100 000 bases or base pairs.

9. The method according to any of claims 1 to 8, wherein the DNA construct which is capable of episomal replication is selected from the group consisting of circular plasmid vectors, phagemids and cosmids.

10. The method according to any of claims 1 to 9, wherein the prokaryotic host cell has at least one further deficiency with regard to a gene which has a function in the metabolization of rhamnose, where said gene encodes a protein selected from the group consisting of rhamnulose 1-phosphatase (RhaB) and rhamnulose-phosphate aldolase (RhaD).

11. The method according to any of claims 1 to 10, wherein the expression of the nucleic acid sequence to be expressed causes the production of a protein encoded by said nucleic acid sequence.

12. The method according to any of claims 1 to 11, wherein the nucleic acid sequence to be expressed encodes a recombinant protein selected from the group consisting of chymosines, proteases, polymerasen, saccharidases, dehydrogenases, nucleases, glucanases, glucose oxidases, α-amylases, oxidoreductases, peroxidases, laccases, xylanases, phytases, cellulases, collagenases, hemicellulases, lipases, lactases, pectinases, amyloglucosidases, glucoamylases, pullulanases, glucose isomerases, nitrilases, esterases, nitrile hydratases, amidases, oxygenases, oxynitrilases, lyases, lactonases, carboxylases, collagenases, cellulases, serum albumins, factor VII, factor VIII, factor IX, factor X, tissue plasminogen factors, protein C, von Willebrand factors, antithrombins, erythropoietins, colony-stimulating factors, cytokins, interleukins, insulins, integrins, addressins, selectins, antibodies, antibody fragments, structural proteins, collagen, fibroins, elastins, tubulins, actins, myosins, growth factors, cell-cycle proteins, vaccines, fibrinogens and thrombins.

13. A prokaryotic host cell which is at least deficient with regard to L-rhamnose isomerase and which comprises at least one DNA construct which is capable of replication in said host cell and which comprises a nucleic acid sequence to be expressed under the transcriptional control of an L-rhamnose-inducible promoter, where said promoter is heterologous with regard to said nucleic acid sequence.

14. The use of a prokaryotic host cell according to claim 13 for the production of foodstuffs, feedstuffs, enzymes, chemicals, pharmaceuticals or fine chemicals.

15. A method for the production of recombinant proteins, enzymes and fine chemicals using a prokaryotic host cell according to claim 13 or a preparation thereof.

## Revendications

1. Procédé pour l'expression de séquences d'acide nucléique dans des cellules hôtes procaryotes, dans lequel :
a) au moins une construction d'ADN, pouvant être répliquée de manière épisomale dans lesdites cellules hôtes et comportant une séquence d'acide nucléique à exprimer sous le contrôle transcriptionnel d'un promoteur pouvant être induit par le L-rhamnose, ledit promoteur étant hétérologue par rapport à ladite séquence d'acide nucléique, est introduite dans lesdites cellules hôtes,
b) des cellules hôtes procaryotes, qui contiennent ladite construction d'ADN sous forme épisomale, sont sélectionnées et
c) l'expression de ladite séquence d'acide nucléique est induite par ajout de L-rhamnose pour donner une culture desdites cellules hôtes sélectionnées,
**caractérisé en ce que** la cellule hôte procaryote est au moins déficiente par rapport à la L-rhamnose-isomérase.

2. Procédé selon la revendication 1, dans lequel la cellule hôte procaryote est choisie parmi les variétés de la famille des entérobactéries ou de l'ordre des actinomycétales.

3. Procédé selon l'une des revendications 1 ou 2, dans lequel la cellule hôte procaryote est *Escherichia coli.*

4. Procédé selon l'une quelconque des revendications 1 à 3, dans lequel le promoteur pouvant être induit par le L-rhamnose est le promoteur rhaP_{BAD} issu *d'E. coli* ou un équivalent fonctionnel de ce même promoteur ou un fragment fonctionnellement équivalent de ceux-ci.

5. Procédé selon l'une quelconque des revendications 1 à 4, dans lequel le promoteur pouvant être induit par le L-rhamnose contient au moins un élément de ligature RhaS selon la séquence SEQ ID N° : 5 ou un équivalent fonctionnel de ce même élément ou un fragment fonctionnellement équivalent de ceux-ci.

6. Procédé selon l'une quelconque des revendications 1 à 5, dans lequel le promoteur pouvant être induit par le L-rhamnose contient au moins une séquence décrite par SEQ ID N° : 1, 2, 3, ou 4.

7. Procédé selon l'une quelconque des revendications 1 à 6, dans lequel la L-rhamnose-isomérase est décrite par la séquence d'acides aminés selon la séquence SEQ ID N° : 9 ou un équivalent fonctionnel de cette même séquence.

8. Procédé selon l'une quelconque des revendications 1 à 7, dans lequel la construction d'ADN pouvant être répliquée de manière épisomale a une taille d'au maximum 100 000 bases ou paires de bases.

9. Procédé selon l'une quelconque des revendications 1 à 8, dans lequel la construction d'ADN pouvant être répliquée de manière épisomale est choisie parmi le groupe constitué des phagemides, des cosmides et des vecteurs de plasmides circulaires.

10. Procédé selon l'une quelconque des revendications 1 à 9, dans lequel la cellule hôte procaryote présente au moins une autre déficience par rapport à un gène ayant une fonction de métabolisation du rhamnose, ledit gène codant pour une protéine choisie parmi le groupe constitué de la rhamnulose-1-phosphatase (RhaB) et de la rhamnulose-phosphate-aldolase (RhaD).

11. Procédé selon l'une quelconque des revendications 1 à 10, dans lequel l'expression de la séquence d'acide nucléique à exprimer conditionne la production d'une protéine codée par ladite séquence d'acide nucléique.

12. Procédé selon l'une quelconque des revendications 1 à 11, dans lequel la séquence d'acide nucléique à exprimer code pour une protéine recombinante choisie dans le groupe constitué des chymosines, des protéases, des polymérases, des saccharidases, des déshydrogénases, des nucléases, des glucanases, des glucose oxydases, des α-amylases, des oxydoréductases, des peroxydases, des laccases, des xylanases, des phytases, des cellulases, des collagénases, des hémicellulases, des lipases, des lactases, des pectinases, des amyloglucosidases, des glucoamylases, des pullulanases, des glucose isomérases, des nitrilases, des estérases, des nitrile hydratases, des amidases, des oxygénases, des oxynitrilases, des lyases, des lactonases, des carboxylases, des collagénases, des cellulases, des albumines sériques, du facteur VII, du facteur VIII, du facteur IX, du facteur X, des facteurs plasminogènes tissulaires, de la protéine C, des facteurs von Willebrand, des antithrombines, des érythropoïétines, "des facteurs de stimulation de colonie", des cytokines, des interleukines, des insulines, de l'intégrine, de l'adressine, des sélectines, des anticorps, des fragments d'anticorps, des protéines structurelles, du collagène, des fibroïnes, des élastines, des tubulines, des actines, des myosines, des facteurs de croissance, des protéines du cycle cellulaire, des inoculants, des fibrinogènes et des thrombines.

13. Cellule hôte procaryote, au moins déficiente par rapport à la L-rhamnose-isomérase et contenant au moins une construction d'ADN pouvant être répliquée dans ladite cellule hôte, laquelle construction comporte une séquence d'acide nucléique à exprimer sous le contrôle transcriptionnel d'un promoteur pouvant être induit par le L-rhamnose, ledit promoteur étant hétérologue par rapport à ladite séquence d'acide nucléique.

14. Utilisation d'une cellule hôte procaryote selon la revendication 13 pour la préparation d'aliments ou de produits de fourrage, d'enzymes, de produits chimiques, de produits pharmaceutiques ou de produits de chimie fine.

15. Procédé de préparation de protéines recombinantes, d'enzymes et de produits de chimie fine par l'utilisation d'une cellule hôte procaryote selon la revendication 13 ou d'une préparation de cette même cellule.
